# EUROPEAN PATENT SPECIFICATION

(11) **EP 4 204 570 B1**
(45) Date of publication and mention of the grant of the patent: **04.06.2025**
(21) Application number: 21790425.9
(22) Date of filing: 08.10.2021
(51) Int. Cl.: C12N 15/63

(54) **METHOD OF PRODUCING A RECOMBINANT PROTEIN IN A HOST CELL WHICH HAS A DISABLED RHAMNOSE METABOLISM AS WELL AS EXPRESSION VECTORS, HOST CELLS AND RECOMBINANT PROTEINS THEREOF**
VERFAHREN ZUR HERSTELLUNG EINES REKOMBINANTEN PROTEINS IN EINER WIRTSZELLE MIT EINEM DEAKTIVIERTEN RHAMNOSEMETABOLISMUS SOWIE EXPRESSIONSVEKTOREN, WIRTSZELLEN UND REKOMBINANTEN PROTEINEN DAVON
PROCÉDÉ DE PRODUCTION D'UNE PROTÉINE RECOMBINANTE DANS UNE CELLULE HÔTE QUI POSSÈDE UN MÉTABOLISME DU RHAMNOSE DÉSACTIVÉ AINSI QUE DES VECTEURS D'EXPRESSION, CELLULES HÔTES ET PROTÉINES RECOMBINANTES ASSOCIÉES

(30) Priority: 09.10.2020 EP 20201096
(43) Date of publication of application: 05.07.2023
(73) Proprietor: STADA ARZNEIMITTEL AG, 61118 Bad Vilbel (DE); Xbrane Biopharma AB, 171 65 Solna (SE)
(72) Inventor: VIKSTRÖM, David, 194 68 Upplands Väsby (SE); ISMAIL, Nurzian, 118 27 Stockholm (SE); SAMUELSON, Patrik, 133 36 Saltsjöbaden (SE); MIRZADEH, Kiavash, 171 52 Solna (SE); GUDISE, Santhosh, 123 48 Farsta (SE); KADOW, Maria, 192 74 Sollentuna (SE); SZEKÉR, Kathleen, 35037 Marburg (DE); SALIH, Tagrid, 168 57 Bromma Kyrka (SE); BARASZKIEWICZ, Mariusz, 168 69 Bromma (SE); BASHIRI, Siavash, 182 39 Danderyd (SE)
(74) Representative: Maiwald GmbH
(86) International application number: PCT/EP2021/077820
(87) International publication number: WO 2022/074182

(56) References cited:
- WO-A1-2012/041899
- WO-A2-2006/061174
- US-A1- 2005 130 205
- KELLY CIARÁN L. ET AL: "Transcriptional Terminators Allow Leak-Free Chromosomal Integration of Genetic Constructs in Cyanobacteria", MICROORGANISMS, vol. 7, no. 8, 16 August 2019 (2019-08-16), pages 263, XP055783447, DOI: 10.3390/microorganisms7080263
- ANNA HJELM ET AL: "-Rhamnose P BAD Promoter-Based Production of Membrane and Secretory Proteins", ACS SYNTHETIC BIOLOGY, vol. 6, no. 6, 3 March 2017 (2017-03-03), Washington, DC,USA, pages 985 - 994, XP055576200, ISSN: 2161-5063, DOI: 10.1021/acssynbio.6b00321
- SANDOMENICO ANNAMARIA ET AL: "Evolution of Escherichia coli Expression System in Producing Antibody Recombinant Fragments", INTERNATIONAL JOURNAL OF MOLECULAR SCIENCES, vol. 21, no. 17, 31 August 2020 (2020-08-31), pages 6324, XP055783684, DOI: 10.3390/ijms21176324
- DEEPAK KUMAR ET AL: "QbD Based Media Development for the Production of Fab Fragments in E. coli", BIOENGINEERING, vol. 6, no. 2, 28 March 2019 (2019-03-28), pages 29, XP055765699, DOI: 10.3390/bioengineering6020029

## Description

### TECHNICAL FIELD

The present invention relates to DNA construct suitable for expressing ranibizumab in a bacterial host cell wherein said DNA construct comprises nucleotide sequences encoding: a *rhaBAD* promoter, a RhaR transcription activator, a RhaS transcription activator, an antibiotic resistance marker, a promoter operably linked to the nucleic acid encoding for the antibiotic resistance marker, an rrnB T1 terminator, an rrnB T2 terminator, and an origin of replication. The DNA construct may also comprise a nucleotide sequence encoding ranibizumab operably linked to the *rhaBAD* promoter. The present invention further relates to a vector and bacterial host cell comprising said DNA construct as well as a method of producing ranibizumab by exposing said bacterial host cell to rhamnose and thereby inducing expression of ranibizumab.

### BACKGRUND OF THE INVENTION

The metabolism of rhamnose involves L-rhamnose being taken up into cells via the permease RhaT and then isomerized into L-rhamnulose by L-rhamnose isomerase (RhaA), and L-rhamnulose is then phosphorylated further by rhamnulokinase (RhaB) and finally hydrolyzed by rhamnulose-1-phosphate aldolase (RhaD) to give dihydroxyacetonephosphate and L-lactaldehyde [1]. The genes *rhaA, rhaB* and *rhaD* form an operon referred to as *rhaBAD* and are transcribed with the aid of the *rhaBAD* promoter [1]. In comparison with other systems, the rhamnose metabolism pathway is distinguished by the fact that two transcription activators known as RhaS and RhaR are required for regulation as explained below [1].

The *rhaBAD* operon is a positively regulated catabolic operon which transcribes above mentioned *rhaB, rhaA* and *rhaD* genes divergently from the *rhaSR* operon with approximately 240 bp of DNA separating their respective transcription start sites [1]. The *rhaSR* operon encodes RhaS and RhaR wherein each monomer of the dimeric RhaS and RhaR proteins contains two helix-turn-helix motifs and contacts two major grooves of DNA. RhaR regulates transcription of *rhaSR* by binding promoter DNA spanning -32 to -82 bases relative to the *rhaSR* transcription start site [1]. Subsequent to *rhaSR* expression, RhaS bind DNA upstream of the *rhaBAD* operon at -32 to -81 bases relative to the transcription start site to increase *rhaBAD* expression [1]. Furthermore, the *rhaSR-rhaBAD* intergenic region contains CRP binding sites at positions -92.5 (CRP 1) relative to the transcription start site of the *rhaBAD* operon and CRP binding sites at positions -92.5 (CRP 2), -115.5 (CRP 3) and -116.5 (CRP 4) relative to the transcription start site of the *rhaSR* operon [1]. The cyclic AMP receptor protein (CRP) regulates the expression of more than 100 promoters in *Escherichia coli.*

DNA constructs comprising DNA sequences encoding RhaS, RhaR and the *rhaBAD* promoter are known in the art. US8138324 discloses pTACO- and pLEMO-derived plasmids (i.e. DNA constructs) comprising DNA sequences encoding RhaS, RhaR and the *rhaBAD* promoter. However, US8138324 is silent about using host cells which have a disabled rhamnose metabolism.

DNA constructs based on pRha-derived plasmids comprising DNA sequences encoding RhaS, RhaR and the *rhaBAD* promoter are also known in the art, for example from Giacalone *et al*. [5] or Hjelm *et al.* [2]. Giacalone *et al.* describe for example the plasmids pRha67A and pRha109A whereas Hjelm *et al.* disclose the plasmid pRha67K.

Sandomenico *et al.* [7] summarize the evolution of *Escherichia coli* expression system in producing antibody recombinant fragments.

Although DNA constructs comprising DNA sequences encoding RhaS, RhaR and the *rhaBAD* promoter are known in the art there are still many challenges, especially in industrial scale production of recombinant proteins, in particular monoclonal antibodies or fragments thereof. The main challenges are:
(i) the host cells being subject to stress which results in damage to cellular macromolecules such as membranes, proteins and nucleic acids;
(ii) poor growth of the host cells;
(iii) poor activity of the produced recombinant proteins; and/or
(iv) obtaining recombinant proteins in low yields.

Accordingly, there is a need for improved DNA constructs as well as a host cell and method suitable for the efficient production of recombinant proteins, such as monoclonal antibodies or fragments thereof in a high yield, particularly when such recombinant proteins are intended for therapeutic applications.

### OBJECTS OF THE INVENTION

Thus, the object of the present invention was to provide improved DNA constructs, as well as a bacterial host cell and method suitable for the efficient production of ranibizumab.

### SUMMARY OF THE INVENTION

A first aspect of the invention relates to a DNA construct for expressing ranibizumab in a bacterial host cell, wherein ranibizumab comprises (i) a light chain comprising the amino acid sequence of SEQ ID 3, and (ii) a heavy chain comprising the amino acid sequence of SEQ ID 4, wherein said DNA construct comprises a nucleotide sequence encoding ranibizumab, wherein said DNA construct further comprises at least one nucleotide sequence encoding a signal peptide which is operably linked in the direction of transcription to the nucleotide sequence encoding the light chain of ranibizumab and/or the heavy chain of ranibizumab, wherein said DNA construct comprises nucleotide sequences encoding:
- *rhaBAD* promoter,
- RhaR transcription activator,
- RhaS transcription activator,
- an antibiotic resistance marker,
- a promoter operably linked to the nucleotide sequence encoding the antibiotic resistance marker
- rrnB T1 terminator
- rrnB T2 terminator, and
- an pMB 1 origin of replication.

In a preferred embodiment, the DNA construct is characterized by:
- the antibiotic resistance marker is a kanamycin resistance marker, preferably a kanamycin resistance marker comprising the nucleotide sequence of SEQ ID 12 or a sequence with at least 90 % sequence identity thereto;
- the promoter operably linked to the nucleotide sequence encoding the antibiotic resistance marker is an AmpR promoter, preferably an AmpR promoter comprising the nucleotide sequence of SEQ ID 13 or a sequence with at least 90 % sequence identity thereto;

- the rrnB T1 terminator comprising the nucleotide sequence of SEQ ID 14 or a sequence with at least 90 % sequence identity thereto;
- the rrnB T2 terminator comprising the nucleotide sequence of SEQ ID 15 or a sequence with at least 90 % sequence identity thereto; and/or
- the pMB 1 origin of replication comprising the nucleotide sequence of SEQ ID 16 or a sequence with at least 90 % sequence identity thereto.

In a preferred embodiment, the DNA construct is characterized by:
- the antibiotic resistance marker being a kanamycin resistance marker comprising the nucleotide sequence of SEQ ID 12;
- the promoter operably linked to the nucleotide sequence encoding the antibiotic resistance marker is an AmpR promoter comprising the nucleotide sequence of SEQ ID 13;
- the rrnB T1 terminator comprising the nucleotide sequence of SEQ ID 14;
- the rrnB T2 terminator comprising the nucleotide sequence of SEQ ID 15; and/or
- the pMB 1 origin of replication comprising the nucleotide sequence of SEQ ID 16.

In a preferred embodiment, the DNA construct is characterized by:
- the *rhaBAD* promoter comprising the nucleotide sequence of SEQ ID 8 or a sequence with at least 90 % sequence identity thereto;
- the RhaR transcription activator comprising the nucleotide sequence of SEQ ID 9 or a sequence with at least 90 % sequence identity thereto;
- the RhaS transcription activator comprising the nucleotide sequence of SEQ ID 11 or a sequence with at least 90 % sequence identity thereto;
- the antibiotic resistance marker is a kanamycin resistance marker comprising the nucleotide sequence of SEQ ID 12 or a sequence with at least 90 % sequence identity thereto;
- the AmpR promoter comprising the nucleotide sequence of SEQ ID 13 or a sequence with at least 90 % sequence identity thereto;
- the rrnB T1 terminator comprising the nucleotide sequence of SEQ ID 14 or a sequence with at least 90 % sequence identity thereto;
- the rrnB T2 terminator comprising the nucleotide sequence of SEQ ID 15 or a sequence with at least 90 % sequence identity thereto; and
- the pMB1 origin of replication comprising the nucleotide sequence of SEQ ID 16 or a sequence with at least 90 % sequence identity thereto.

In a preferred embodiment, the DNA construct is characterized by:
- the *rhaBAD* promoter comprising the nucleotide sequence of SEQ ID 8;
- the RhaR transcription activator comprising the nucleotide sequence of SEQ ID 9;
- the RhaS transcription activator comprising the nucleotide sequence of SEQ ID 11;
- the antibiotic resistance marker is a kanamycin resistance marker comprising the nucleotide sequence of SEQ ID 12;
- the AmpR promoter comprising the nucleotide sequence of SEQ ID 13;
- the rrnB T1 terminator comprising the nucleotide sequence of SEQ ID 14;
- the rrnB T2 terminator comprising the nucleotide sequence of SEQ ID 15; and
- the pMB1 origin of replication comprising the nucleotide sequence of SEQ ID 16.

In a preferred embodiment, the DNA construct comprises a multiple cloning site comprising 17 restriction sites cleavable by restriction enzymes EcoRI, NdeI, NotI, XhoI, PspXI, PaeR71, BbsI, StyI, AvrII, BanI, Acc65I, KpnI, Eco53kI, SacI, BamHI, XbaI, SalI, AccI, PstI, SbfI, SphI and HindIII.

In a preferred embodiment, the DNA construct comprises the nucleotide sequence of SEQ ID 1, or a sequence with at least 90 % sequence identity thereto, preferably the nucleotide sequence of SEQ ID 1.

In a preferred embodiment, the nucleotide sequence encoding ranibizumab is operably linked to the *rhaBAD* promoter.

In a preferred embodiment, the nucleotide sequence encoding ranibizumab comprises (i) a nucleotide sequence encoding for the light chain of ranibizumab comprising the sequence of SEQ ID 5 or a sequence with at least 90 % sequence identity thereto, and/or (ii) a nucleotide sequence encoding for the heavy chain of ranibizumab comprising the sequence of SEQ ID 6 or a sequence with at least 90 % sequence identity thereto. In a further preferred embodiment, said nucleotide sequence encoding ranibizumab comprises (i) a nucleotide sequence encoding for the light chain of ranibizumab comprising the sequence of SEQ ID 5, and/or (ii) a nucleotide sequence encoding for the heavy chain of ranibizumab comprising the sequence of SEQ ID 6.

In an embodiment, the nucleotide sequence encoding the signal peptide is operably linked in the direction of transcription to either one or both of the nucleotide sequence of SEQ ID 5 and SEQ ID 6.

In an embodiment, the signal peptide is PelB (pectate lyase B). In a preferred embodiment, the nucleotide sequence encoding the PelB signal peptide comprises a sequence of SEQ ID 7 or a sequence with at least 90 % sequence identity thereto; more preferably the nucleotide sequence encoding the PelB signal peptide comprises the sequence of SEQ ID 7.

In one embodiment, the resulting PelB signal peptide comprises an amino acid sequence of SEQ ID 18 [6]: MKYLLPTAAAGLLLLAAQPAMA.

In a preferred embodiment, the DNA construct comprises the sequence of SEQ ID 17 or a sequence with at least 90 % sequence identity thereto, preferably comprises the sequence of SEQ ID 17.

A second aspect of the invention relates to an expression vector comprising any of the DNA constructs according to the first aspect of the invention.

A third aspect of the invention relates to a bacterial host cell comprising a DNA construct according to the first aspect of the invention or the expression vector according to the second aspect of the invention, wherein said bacterial host cell is preferably an *Escherichia coli* cell, more preferably an *E. coli* K-12 cell.

In a preferred embodiment, said bacterial host cell either comprises (i) a chromosome which comprises a mutation in the nucleic acid sequence of the *rhaB* gene which renders RhaB inactive, or (ii) a chromosome in which the nucleotide sequence encoding RhaB is deleted.

In a preferred embodiment, said bacterial host cell is an *Escherichia coli* W3110 cell, preferably comprising a chromosome which comprises a frame shift-mutation in the nucleotide sequence encoding RhaB.

In a preferred embodiment, said bacterial host cell is an *Escherichia coli* W3110 cell comprising a chromosome which comprises nucleotide sequence of SEQ ID 2 or a sequence with at least 90 % sequence identity thereto, preferably the nucleotide sequence of SEQ ID 2, wherein optionally the chromosome of the bacterial host cell further comprises a nucleotide sequence encoding RhaT.

A fourth aspect of the invention relates to a method of producing ranibizumab comprising the step of exposing the bacterial host cell according to the third aspect of the invention to rhamnose, thereby inducing expression of ranibizumab. In a preferred embodiment, the method further comprises the step of recovering ranibizumab from the bacterial host cell; and optionally further comprises one or more step(s) of purifying the recovered ranibizumab, preferably by one or more chromatography steps.

One or more of the above indicated SEQ IDs 1, 2, and 5-18 of the various aspects of the invention (and embodiments thereof) may be replaced by a sequence with at least 90 % sequence identity thereto. The term "sequence identity" as used herein is used with regard to amino acid or nucleotide sequences and the sequence identity is over the entire length of the specified sequence. A sequence may thus be at least 90 percent, at least 92 percent, at least 95 percent, at least 96 percent, at least 97 percent, at least 98 percent or at least 99 percent, identical in sequence to the amino acid or nucleotide sequence specified. Such sequences of the invention thus include single or multiple nucleotide or amino acid alterations (additions, substitutions, insertions or deletions) to the sequences of the invention. At the amino acid level preferred sequences with the above defined sequence identity contain up to 5, e.g. only 1, 2, 3, 4 or 5, preferably 1, 2 or 3, more preferably 1 or 2, altered amino acids in the sequences of the invention.

### BRIEF DESCRIPTION OF DRAWINGS

Figure 1 - Plasmid map for KTXHIS

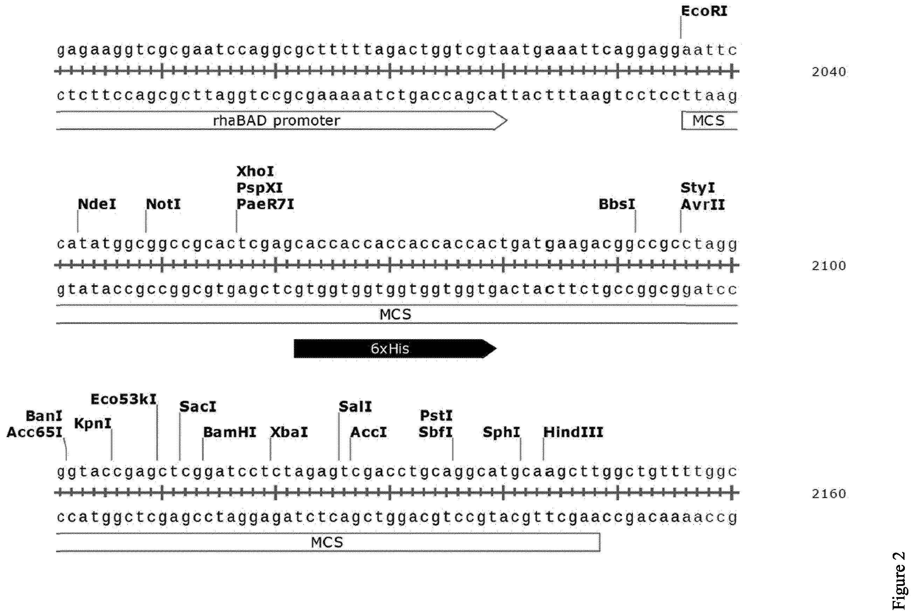

Figure 2 - The nucleotide sequence of the multiple cloning site (MCS) of KTXHIS
Figure 3 - Plasmid map of KTXHIS-PelbLC-PelbHC
Figure 4a-4d - Western blots using anti-FAB antibody - Titratability comparison in small scale experiments.
Figures 5 - Small-scale batch purification from clarified homogenized lysate samples from harvested material with Capto L resin Western blot using anti-FAB antibody. Gel loading: 3 µg product applied except for XB17/67A (only 0.2 µg product applied due to low yield). Marker from 18.4-116 kDa.
Figure 6 - Summary of Affinity HPLC titer data
Figures 7a-7c - Western blots using anti-FAB antibody - Control of expression comparison in small scale experiment.

### DETAILED DESCRIPTION

The present invention relates to regulating the L-rhamnose *rhaBAD* promoter-based production of ranibizumab.

More specifically, the present invention relates to producing ranibizumab comprising the steps of:
a. cloning an a nucleotide sequence encoding ranibizumab into a DNA construct such that the nucleotide sequence is operably linked to a *rhaBAD* promoter, and
b. introducing the resulting nucleotide sequence into a bacterial host cell comprising a chromosome which comprises a mutation or modification which disables rhamnose metabolism.

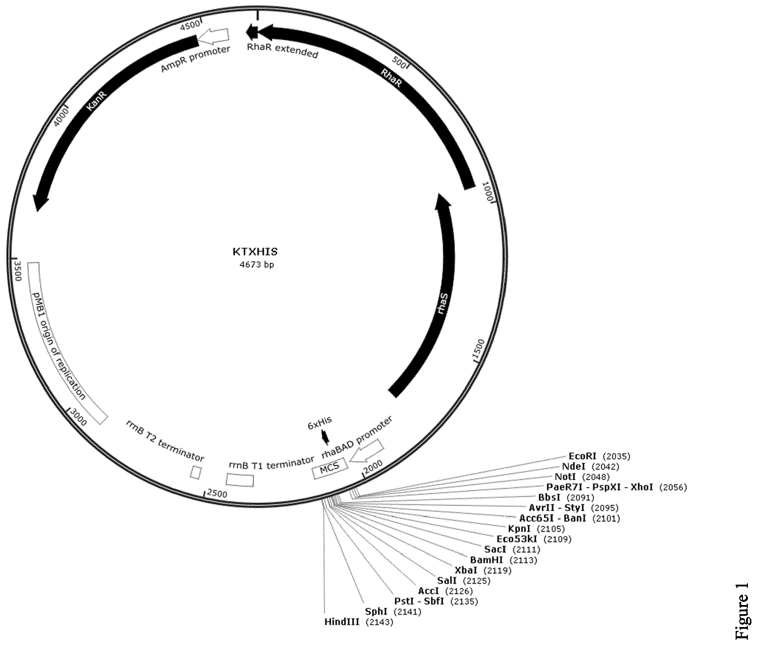

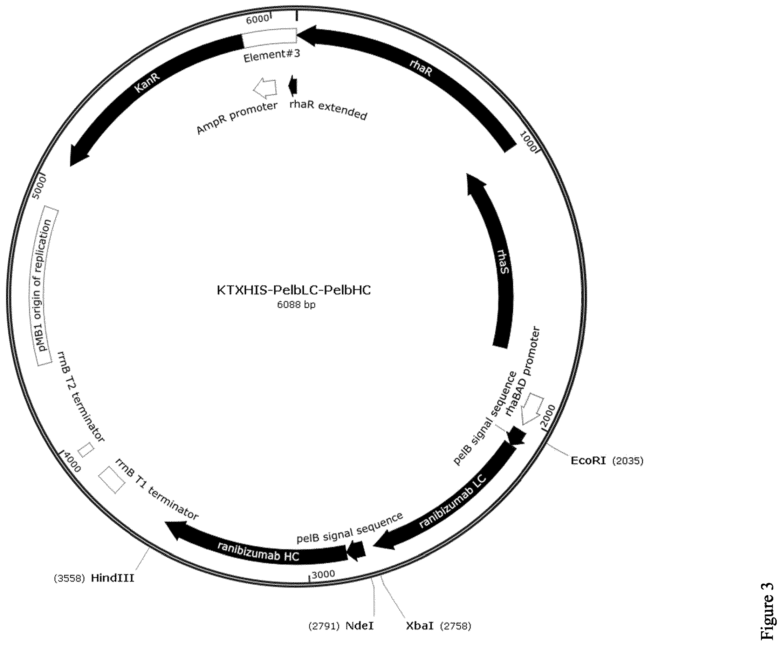

The DNA construct disclosed in the above method comprises a nucleotide sequence encoding the *rhaBAD* promoter. In an embodiment of the invention, the nucleotide sequence of the *rhaBAD* promoter comprises the sequence of SEQ ID 8 (and wherein the sequence is referred to as "rhaBAD" in figures 1 and 3):

CACCACAATTCAGCAAATTGTGAACATCATCACGTTCATCTTTCCCTGGTTGCCAA
TGGCCCATTTTCTTGTCAGTAACGAGAAGGTCGCGAATCCAGGCGCTTTTTAGAC
TGGTCGTA.

The DNA construct comprises a nucleotide sequence encoding the RhaR transcription activator. In an embodiment of the invention, the nucleotide sequence of the RhaR transcription activator comprises a sequence of SEQ ID 9 (and wherein the sequence is referred to as "rhaR" in figures 1 and 3):

ATGGCTTTCTGCAATAACGCGAATCTTCTCAACGTATTTGTACGCCATATTGCGAA
TAATCAACTTCGTTCTCTGGCCGAGGTAGCCACGGTGGCGCATCAGTTAAAACTT
CTCAAAGATGATTTTTTTGCCAGCGACCAGCAGGCAGTCGCTGTGGCTGACCGTT
ATCCGCAAGATGTCTTTGCTGAACATACACATGATTTTTGTGAGCTGGTGATTGTC
TGGCGCGGTAATGGCCTGCATGTACTCAACGATCGCCCTTATCGCATTACCCGTG
GCGATCTCTTTTACATTCATGCTGATGATAAACACTCCTACGCTTCCGTTAACGAT
CTGGTTTTGCAGAATATTATTTATTGCCCGGAGCGTCTGAAGCTGAATCTTGACTG
GCAGGGGGCGATTCCGGGATTTAACGCCAGCGCAGGGCAACCACACTGGCGCTT
AGGTAGCATGGGGATGGCGCAGGCGCGGCAGGTTATTGGTCAGCTTGAGCATGA
AAGTAGTCAGCATGTGCCGTTTGCTAACGAAATGGCTGAGTTGCTGTTCGGGCAG
TTGGTGATGTTGCTGAATCGCCATCGTTACACCAGTGATTCGTTGCCGCCAACATC
CAGCGAAACGTTGCTGGATAAGCTGATTACCCGGCTGGCGGCTAGCCTGAAAAGT
CCCTTTGCGCTGGATAAATTTTGTGATGAGGCATCGTGCAGTGAGCGCGTTTTGC
GTCAGCAATTTCGCCAGCAGACTGGAATGACCATCAATCAATATCTGCGACAGGT
CAGAGTGTGTCATGCGCAATATCTTCTCCAGCATAGCCGCCTGTTAATCAGTGAT
ATTTCGACCGAATGTGGCTTTGAAGATAGTAACTATTTTTCGGTGGTGTTTACCCG
GGAAACCGGGATGACGCCCAGCCAGTGGCGTCATCTCAATTCGCAGAAAGAT.

The DNA construct may further comprise a nucleotide sequence encoding an extension of the RhaR transcription activator which is in frame with RhaR because of a missing stop codon. In an embodiment of the invention, the nucleotide sequence of the extension of the RhaR transcription activator comprises the sequence of SEQ ID 10 (and wherein the sequence is referred to as "rhaR extended" in figures 1 and 3):
AGACGAAAGGGCCTCGTGATACGCCTATTTTTATAG.

The DNA construct comprises a nucleotide sequence encoding the RhaS transcription activator. In an embodiment of the invention, the nucleotide sequence of the RhaS transcription activator comprises the sequence of SEQ ID 11 (and wherein the sequence is referred to as "rhaS" in figures 1 and 3):

ATGACCGTATTACATAGTGTGGATTTTTTCCGTCTGGTAACGCGTCCGTGGCGAT
AGAACCCCGGCTCCCGCAGGCGGATTTCCTGAACATCATCATGATTTCATGAA

ATTGTGATTGTCGAACATGGCACGGGTATTCATGTGTTTAATGGGCAGCCCTATA
CCATCACCGGTGGCACGGTCTGTTTCGTACGCGATCATGATCGGCATCTGTATGA
ACATACCGATAATCTGTGTCTGACCAATGTGCTGTATCGCTCGCCGGATCGATTTC
AGTTTCTCGCCGGGCTGAATCAGTTGCTGCCACAAGAGCTGGATGGGCAGTATCC
GTCTCACTGGCGCGTTAACCACAGCGTATTGCAGCAGGTGCGACAGCTGGTTGCA
CAGATGGAACAGCAGGAAGGGGAAAATGATTTACCCTCGACCGCCAGTCGCGAG
ATCTTGTTTATGCAATTACTGCTCTTGCTGCGTAAAAGCAGTTTGCAGGAGAACCT
GGAAAACAGCGCATCACGTCTCAACTTGCTTCTGGCCTGGCTGGAGGACCATTTT
GCCGATGAGGTGAATTGGGATGCCGTGGCGGATCAATTTTCTCTTTCACTGCGTA
CGCTACATCGGCAGCTTAAGCAGCAAACGGGACTGACGCCTCAGCGATACCTGA
ACCGCCTGCGACTGATGAAAGCCCGACATCTGCTACGCCACAGCGAGGCCAGCG
TTACTGACATCGCCTATCGCTGTGGATTCAGCGACAGTAACCACTTTTCGACGCTT
TTTCGCCGAGAGTTTAACTGGTCACCGCGTGATATTCGCCAGGGACGGGATGGCT
TTCTGCAATAA.

The DNA construct comprises a nucleotide sequence encoding an "antibiotic resistance marker" or "selection marker". Such a marker is a fragment of DNA that contains a gene whose product confers resistance to an antibiotic (e.g., chloramphenicol, ampicillin, gentamycin, streptomycin, tetracycline, kanamycin, neomycin) or the ability to grow on selective media (e.g., ura (uracil), leu (leucine), trp (tryptophan), his (histidine)). Usually, plasmids contain antibiotic resistance marker to force the bacterial cell to maintain the plasmid. In an embodiment of the invention, the DNA construct may comprise a nucleotide sequence of a kanamycin resistance marker. In a specific embodiment of the invention, the nucleotide sequence for conferring kanamycin resistance comprises the sequence of SEQ ID 12 (and wherein the sequence is referred to as "KanR" in figures 1 and 3):

ATGAGCCATATTCAACGGGAAACGTCTTGCTCTAGGCCGCGATTAAATTCCAACA
TGGATGCTGATTTATATGGGTATAAATGGGCTCGCGATAATGTCGGGCAATCAGG
TGCGACAATCTATCGATTGTATGGGAAGCCCGATGCGCCAGAGTTGTTTCTGAAA
CATGGCAAAGGTAGCGTTGCCAATGATGTTACAGATGAGATGGTCAGACTAAACT
GGCTGACGGAATTTATGCCTCTTCCGACCATCAAGCATTTTATCCGTACTCCTGAT
GATGCATGGTTACTCACCACTGCGATCCCCGGGAAAACAGCATTCCAGGTATTAG
AAGAATATCCTGATTCAGGTGAAAATATTGTTGATGCGCTGGCAGTGTTCCTGCG
CCGGTTGCATTCGATTCCTGTTTGTAATTGTCCTTTTAACAGCGATCGCGTATTTC

GTCTCGCTCAGGCGCAATCACGAATGAATAACGGTTTGGTTGATGCGAGTGATTT
TGATGACGAGCGTAATGGCTGGCCTGTTGAACAAGTCTGGAAAGAAATGCATAA
ACTTTTGCCATTCTCACCGGATTCAGTCGTCACTCATGGTGATTTCTCACTTGATA
ACCTTATTTTTGACGAGGGGAAATTAATAGGTTGTATTGATGTTGGACGAGTCGG
AATCGCAGACCGATACCAGGATCTTGCCATCCTATGGAACTGCCTCGGTGAGTTT
TCTCCTTCATTACAGAAACGGCTTTTTCAAAAATATGGTATTGATAATCCTGATAT
GAATAAATTGCAGTTTCATTTGATGCTCGATGAGTTTTTCTAA.

The DNA construct comprises a nucleotide sequence encoding a promoter operably linked to the nucleic acid sequence encoding the antibiotic resistance marker. Such a promotor may increase the expression of the antibiotic resistance markers discussed in the previous paragraph. In an embodiment of the invention, the promoter for ampicillin resistance is an AmpR promoter which is not only capable of promoting expression of ampicillin resistance markers but also capable of promoting expression of kanamycin resistance markers. In a specific embodiment of the invention, the nucleic acid sequence of the AmpR promoter comprises the sequence of SEQ ID 13 (and wherein the sequence is referred to as "AmpR promoter" in figures 1 and 3):

CGCGGAACCCCTATTTGTTTATTTTTCTAAATACATTCAAATATGTATCCGCTCAT
GAGACAATAACCCTGATAAATGCTTCAATAATATTGAAAAAGGAAGAGT

The DNA construct comprises a nucleotide sequence encoding for both of the rrnB T1 terminator and the rrnB T2 terminator. The rrnB T1 and T2 terminators are both efficient transcription terminators in isolated forms, however, when used together, rrnB T1 and T2 terminators may more efficiently terminate transcription.

In a specific embodiment of the invention, the nucleotide sequence of the rrnB T1 terminator comprises the sequence of SEQ ID 14:

CAAATAAAACGAAAGGCTCAGTCGAAAGACTGGGCCTTTCGTTTTATCTGTTGTT
TGTCGGTGAACGCTCTCCTGAGTAGGACAAAT

In a specific embodiment of the invention, the nucleotide sequence of the rrnB T2 terminator comprises the sequence of SEQ ID 15:
AGAAGGCCATCCTGACGGATGGCCTTTT

The DNA construct further comprises a pMB1 origin of replication which is a particular nucleotide sequence at which DNA replication is initiated. DNA replication may proceed from this point bidirectionally or unidirectionally. In a specific embodiment of the invention, the nucleic acid sequence of pMB 1comprises the sequence of SEQ ID 16:

TTTCCATAGGCTCCGCCCCCCTGACGAGCATCACAAAAATCGACGCTCAAGTCAG
AGGTGGCGAAACCCGACAGGACTATAAAGATACCAGGCGTTTCCCCCTGGAAGC
TCCCTCGTGCGCTCTCCTGTTCCGACCCTGCCGCTTACCGGATACCTGTCCGCCTT
TCTCCCTTCGGGAAGCGTGGCGCTTTCTCATAGCTCACGCTGTAGGTATCTCAGTT
CGGTGTAGGTCGTTCGCTCCAAGCTGGGCTGTGTGCACGAACCCCCCGTTCAGCC
CGACCGCTGCGCCTTATCCGGTAACTATCGTCTTGAGTCCAACCCGGTAAGACAC
GACTTATCGCCACTGGCAGCAGCCACTGGTAACAGGATTAGCAGAGCGAGGTAT
GTAGGCGGTGCTACAGAGTTCTTGAAGTGGTGGCCTAACTACGGCTACACTAGAA
GGACAGTATTTGGTATCTGCGCTCTGCTGAAGCCAGTTACCTTCGGAAAAAGAGT
TGGTAGCTCTTGATCCGGCAAACAAACCACCGCTGGTAGCGGTGGTTTTTTTGTTT
GCAAGCAGCAGATTACGCGCAGAAAAAAAGGATCTCAA

In an alternative embodiment of the invention, the DNA construct is an expression plasmid which comprises a nucleotide sequence encoding one or more of:
- *rhaBAD* promoter,
- RhaR transcription activator,
- RhaS transcription activator,
- an antibiotic resistance marker,
- a promoter operably linked to the nucleic acid encoding the antibiotic resistance marker,
- rrnB T1 terminator,
- rrnB T2 terminator, and
- a pMB 1 origin of replication,
wherein the DNA construct optionally comprises a multiple cloning site comprising 17 restriction sites cleavable by restriction enzymes EcoRI, NdeI, NotI, XhoI, PspXI, PaeR71, BbsI, StyI, AvrII, BanI, Acc65I, KpnI, Eco53kI, SacI, BamHI, XbaI, SalI, AccI, PstI, SbfI, SphI and HindIII.

In an embodiment of the invention, the above disclosed expression plasmid comprises a nucleotide sequence encoding:
- *rhaBAD* promoter,
- RhaR transcription activator,
- RhaS transcription activator,
- an antibiotic resistance marker,
- a promoter operably linked to the nucleic acid encoding the antibiotic resistance marker,
- rrnB T1 terminator,
- rrnB T2terminator, and
- a pMB1 origin of replication.

In an embodiment of the invention, the above disclosed expression plasmid comprises a nucleotide sequence encoding:
- *rhaBAD* promoter,
- RhaR transcription activator,
- RhaS transcription activator,
- a kanamycin resistance marker,
- AmpR promoter operably linked to the nucleotide sequence encoding the kanamycin resistance marker,
- rrnB T1 terminator,
- rrnB T2terminator, and
- pMB1 origin of replication.

In an embodiment of the invention, the above disclosed expression plasmid comprises nucleotide sequences encoding:
- *rhaBAD* promoter comprising the nucleotide sequence of SEQ ID 8,
- RhaR transcription activator comprising the nucleotide sequence of SEQ ID 9,
- RhaS transcription activator comprising the nucleotide sequence of SEQ ID 11,
- a kanamycin resistance marker comprising the nucleotide sequence of SEQ ID 12,
- AmpR promoter comprising the nucleotide sequence of SEQ ID 13,
- rrnB T1 terminator comprising the nucleotide sequence of SEQ ID 14,
- rrnB T2 terminator comprising the nucleotide sequence of SEQ ID 15, and
- pMB1 origin of replication comprising the nucleotide sequence of SEQ ID 16.

In an embodiment of the invention, the DNA construct is a plasmid which comprises the nucleotide sequence of SEQ ID 1 and wherein said DNA construct is also referred to as KTXHIS in the present invention.

The nucleotide sequence encoding ranibizumab comprises a nucleic acid encoding the heavy and light chains of ranibizumab. Ranibizumab is a monoclonal antibody fragment (Fab) which is an anti-angiogenic that has been approved to treat the "wet" type of age-related macular degeneration, a common form of age-related vision loss.

In a specific embodiment of the invention, the nucleotide sequence encoding the heavy chain of ranibizumab comprises the sequence of SEQ ID 6:

GAAGTTCAGCTGGTTGAAAGCGGTGGTGGTCTGGTTCAGCCTGGTGGTAGCCTGC
GTCTGAGCTGTGCAGCAAGCGGTTATGATTTTACCCATTATGGTATGAATTGGGTT
CGTCAGGCACCGGGTAAAGGTCTGGAATGGGTTGGTTGGATTAATACCTATACCG
GTGAACCGACCTATGCAGCAGATTTTAAACGTCGTTTTACCTTTAGCCTGGATACC
AGCAAAAGCACCGCATATCTGCAGATGAATAGCCTGCGTGCAGAGGATACCGCA
GTGTATTATTGTGCAAAATATCCGTATTATTACGGCACCAGCCATTGGTATTTCGA
TGTTTGGGGTCAGGGCACCCTGGTTACCGTTAGCAGCGCAAGCACCAAAGGTCCG
AGCGTTTTTCCGCTGGCACCGAGCAGCAAAAGTACCAGCGGTGGCACCGCAGCA
CTGGGTTGTCTGGTTAAAGATTATTTTCCGGAACCGGTTACCGTGAGCTGGAATA
GCGGTGCACTGACCAGCGGTGTTCATACCTTTCCGGCAGTTCTGCAGAGCAGCGG
TCTGTATAGCCTGAGCAGCGTTGTTACCGTTCCGAGCAGCAGCCTGGGCACCCAG
ACCTATATTTGTAATGTTAATCATAAACCGAGCAATACCAAAGTGGATAAAAAAG
TGGAACCGAAAAGCTGCGATAAAACCCATCTGTAA.

In a specific embodiment of the invention, the nucleotide sequence encoding the light chain of ranibizumab comprises the sequence of SEQ ID 5:

GATATTCAGCTGACCCAGAGCCCGAGCAGCCTGAGCGCAAGCGTTGGTGATCGTG
TTACCATTACCTGTAGCGCAAGCCAGGATATTAGCAATTATCTGAATTGGTATCA
GCAGAAACCGGGTAAAGCACCGAAAGTGCTGATCTATTTTACCAGCAGCCTGCAT

AGCGGTGTTCCGAGCCGTTTTAGCGGTAGCGGTAGTGGCACCGATTTTACCCTGA
CCATTAGCAGCCTGCAGCCGGAAGATTTTGCAACCTATTATTGTCAGCAGTATAG
CACCGTTCCGTGGACCTTTGGTCAGGGCACCAAAGTTGAAATTAAACGTACCGTT
GCAGCACCGAGCGTTTTTATCTTTCCGCCTAGTGATGAACAGCTGAAAAGCGGCA
CCGCAAGCGTTGTTTGTCTGCTGAATAACTTTTATCCGCGTGAAGCAAAAGTTCA
GTGGAAAGTTGATAATGCACTGCAGAGCGGTAATAGCCAAGAAAGCGTTACCGA
ACAGGATAGCAAAGATAGCACCTATAGCCTGAGCAGCACCCTGACCCTGAGCAA
AGCAGATTATGAAAAACACAAAGTGTATGCCTGCGAAGTTACCCATCAGGGTCTG
AGCAGTCCGGTTACCAAAAGTTTTAATCGTGGTGAATGCTAA.

The nucleic acid encoding the heavy and light chains of ranibizumab further comprises a nucleotide sequence encoding a signal peptide operably linked to either one or both of the nucleotide sequences encoding the heavy and light chains of ranibizumab. The nucleic acid sequence encoding the signal peptide may be a nucleotide sequence encoding the PelB signal peptide. In a specific embodiment of the invention, the nucleotide sequence encoding the PelB signal peptide comprises the sequence of SEQ ID 7:

ATGAAATATCTGCTGCCGACCGCAGCAGCGGGTCTGCTGCTGCTGGCAGCACAGC
CTGCAATGGCA.

The resulting nucleotide sequence (i.e. the product of cloning a nucleotide sequence encoding ranibizumab into a DNA construct) is preferably the nucleotide sequence of SEQ ID 17 which is also referred to as KTXHIS-PelbLC-PelbHC in the present invention.

The bacterial host cell to be used for producing ranibizumab comprises a chromosome having a mutation or modification which disables rhamnose metabolism. The bacterial host cell may be an *E. coli* cell. In a preferred embodiment, the bacterial host cell is an *E. coli* K-12 cell, more preferably the bacterial host cell is an *E. coli* W3110 cell. The disabled rhamnose metabolism is achieved by a mutation in the nucleotide sequence encoding RhaB which renders RhaB inactive. Alternatively, the disabled rhamnose metabolism is achieved by using a bacterial host cell having a chromosome in which the nucleotide sequence encoding RhaB is deleted; this can e.g. be achieved by deleting the nucleotide sequence encoding RhaB. Preferably, the chromosome of the bacterial host cell comprises the nucleic acid sequence encoding RhaT, i.e. the RhaT gene is intact.

In an embodiment of the invention, the disabled rhamnose metabolism is achieved by a frame shift-mutation in the nucleotide sequence encoding RhaB. Such a mutation results in a bacterial host cell having a chromosome which comprises the nucleotide sequence of SEQ ID 2:

GTTGGCCCCGCTCCACGCCAGTAGCGACTCGTCCCAGTCGTCGCTATTGATATTG
ACCAGTTGCGTGGTCGTGGCGTTGGTATATTCCCAGTTCATCTTGCCGGTCAGGCG
ATAACTGAAGTAATCCGGCATCAGCAGAGCGTGAGCAATGTGTGGAATAAGTTC
AGGTTGTTGCTCCGTCAGCGCACGCAACTGATAAAGCGTATTGAAGGGCAGAAA
CTGGATGCCGCTACGTTGATAAATATCGCGTTTGCCGAGTTGTTGTGCCTGCG
CCATTAGGCCATTGGTGCGGCTATCGCGATAAGCAACGGGCAGGCCCACACGCTG
ACCCTGTTGGTCGAGCAGCACAAAGTCCACGCCCCAGGTATCAATCCCAATGCTA
TCGCGATACGAATCCCTTCCTCGCACACCTTGTTTAATCCAAGGCGAATGGCACTT
TCCAGGCTATCCACATCCCAGGTGACATAGCCGTTCTGACTATGCAGCCCATTGTT
AAAACGATGGATTTCGCGCAGCGTCAGGCTGCGGCATTCACGCTCGTAACGCGCC
AGCATCACGCGCCCACTGGATGCGCCGAGATCGACGGCGACACAATTGCGAAAG
GTCATAATGTGATCCTGCTGAATTTCATTACGACCAGTCTAAAAAGCGCCTGAAT
TCGCGACCTTCTCGTTACTGACAGGAAAATGGGCCATTGGCAACCAGGGAAAGAT
GAACGTGATGATGTTCACAATTTGCTGAATTGTGGTGATGTGATGCTCACC.

The bacterial host cell having a chromosome which comprises the nucleotide sequence of SEQ ID 2 is in the present invention referred to as XdevK.

In one embodiment of the invention the method for developing the XdevK host cell comprises the steps of:
a) constructing the pRhaBFS plasmid, and
b) constructing of the RhaB frame shift mutant.

For constructing the pRhaBFS plasmid the two primers to PCR amplify parts of the *rhaBAD* operon from the chromosome of the *E. coli* BL21 strain (names of primers in parenthesis) are preferably:
- (SBamHI1680 - SEQ ID 19) 5' GTACGCTAGGATCCTGTGGCAGCAACTGATTC 3'
- (BSalI1681 - SEQ ID 20) 5' GTAGATAGGTCGACAGCGATCGTCATTGGGAT 3'

The present invention has multiple aspects, illustrated in examples 1-8 in the below nonlimiting EXAMPLES section.

### EXAMPLES

Example 1 relates to the construction of the XdevK host cell.

Example 2 relates to the construction of the KTXHIS-PelbLC-PelbHC expression vector.

Example 3 relates to the light and the heavy chains of ranibizumab expressed by the expression vector in example 2.

Example 4 shows the superior titer of ranibizumab produced according to the present invention when compared to the titer of ranibizumab produced by the method described in a prior art document.

Example 5 shows that ranibizumab produced according to the present invention is free of bacteriophages.

Example 6 is a comparative experiment showing superior titratability of the present invention when compared to prior art host cells and/or plasmids.

Example 7 is a comparative experiment showing superior quality, purity and titer of the present invention when compared to prior art host cells and/or plasmids.

Example 8 is a comparative experiment showing superior control of expression of the present invention when compared to prior art host cells and/or plasmids.

### Example 1 - Strain development

The XdevK host cell is an *E. coli* W3310 derivative with a frameshift in the chromosomal copy of RhaB making it unable to utilize rhamnose as a carbon source. The XdevK host cell was developed as a strain for a rhamnose inducible system where the nucleotide sequence encoding ranibizumab is cloned into the KTXHIS plasmid and expressed under the control of a rhamnose inducible promoter.

The starting strain for the XdevK host cell was *E. coli* W3110 and a similar method of producing the XdevK host cell is described in Wilms et al. in the section titled "Construction of the Production Strain E. coli BW3110" [3].

The method for developing the XdevK host cell comprises the steps of:
c) constructing the pRhaBFS plasmid, and
d) constructing of the RhaB frame shift mutant.

### Step (a) - Construction of the pRhaBFS plasmid:

The following two primers were used to PCR amplify parts of the *rhaBAD* operon from the chromosome of the *E. coli* BL21 strain (names of primers in parenthesis):
- (SBamHI1680) 5' GTACGCTAGGATCCTGTGGCAGCAACTGATTC 3'
- (BSalI1681) 5' GTAGATAGGTCGACAGCGATCGTCATTGGGAT 3'

The PCR product was purified and digested with BamHI and SalI and ligated into a pLemo plasmid (such as pLemo described in US8138324) which had been digested with BamHI and SalI. The pLemo plasmid with the insert was then digested with ClaI. The digest was incubated with T4 polymerase to make blunt ends and then ligated. The resulting pLemo plasmid with the inserted *rhaBAD* operon genomic region with two additional bases was incubated with BamHI and SalI. The fragment containing the *rhaBAD* operon genomic region with 2 additional bases was purified and ligated with the pmak705 plasmid digested with BamHI and SalI. The plasmid resulting from the ligation was named pRhaBFS and the sequence was verified by sequencing.

### Step (b) - Construction of the RhaB frame shift mutant.

*E. coli* W3110 cells were transformed with the gene replacement plasmid pRhaBFS and then plated on LB agar vegitone containing chloramphenicol (20µg/ml) and incubated at 30 °C for 20 hours. A single colony was picked and placed in LB vegitone and grown in 30° C until the OD₆₀₀ reached 0.5. 5 µl were transferred from the culture to a LB agar vegitone plate containing chloramphenicol (20µg/ml) and incubated at 43 °C for 16 hours. Colonies were tested for correct insert into the chromosome using PCR. Colonies with correct insert were grown in LB vegitone containing chloramphenicol (20µg/ml) to saturation three times and then grow 20 hours in LB vegitone. One µl was then plated onto a LB vegitone plate. The LB vegitone plate was used as a template for replica plating onto a McConkey agar plate supplemented with 1% Rhamnose to verify that the cells cannot utilize Rhamnose as a carbon source. One colony was picked and grown in LB vegitone (Sigma) and then aliquoted and named XdevK expression strain. The chromosomal copy of the *rhaB* gene from XdevK was amplified by means of PCR. The PCR product was sequenced and the correct insertion of two bases (CG) was confirmed (see underlined CG bases in SEQ ID 2):

GTTGGCCCCGCTCCACGCCAGTAGCGACTCGTCCCAGTCGTCGCTATTGATATTG
ACCAGTTGCGTGGTCGTGGCGTTGGTATATTCCCAGTTCATCTTGCCGGTCAGGCG
ATAACTGAAGTAATCCGGCATCAGCAGAGCGTGAGCAATGTGTGGAATAAGTTC
AGGTTGTTGCTCCGTCAGCGCACGCAACTGATAAAGCGTATTGAAGGGCAGAAA
CTGGATGCCGCTACGTTGATAAATATCGCGTTTGCCGAGTTGTTGTTGTGCCTGCG
CCATTAGGCCATTGGTGCGGCTATCGCGATAAGCAACGGGCAGGCCCACACGCTG
ACCCTGTTGGTCGAGCAGCACAAAGTCCACGCCCCAGGTATCAATCCCAATGCTA
TCGCGATACGAATCCCTTCCTCGCACACCTTGTTTAATCCAAGGCGAATGGCACTT
TCCAGGCTATCCACATCCCAGGTGACATAGCCGTTCTGACTATGCAGCCCATTGTT
AAAACGATGGATTTCGCGCAGCGTCAGGCTGCGGCATTCACGCTCGTAACGCGCC
AGCATCACGCGCCCACTGGATGCGCCGAGATCGACGGCGACACAATTGCGAAAG
GTCATAATGTGATCCTGCTGAATTTCATTACGACCAGTCTAAAAAGCGCCTGAAT
TCGCGACCTTCTCGTTACTGACAGGAAAATGGGCCATTGGCAACCAGGGAAAGAT
GAACGTGATGATGTTCACAATTTGCTGAATTGTGGTGATGTGATGCTCACC

### Example 2 - KTXHIS-PelbLC-PelbHC

The PelB signal peptide comprising the nucleotide sequence of SEQ ID 7 was fused in front of nucleic acids encoding both the light and the heavy chains of ranibizumab and then cloned into the KTXHIS expression plasmid. The resulting nucleotide sequence comprises a nucleotide sequence of SEQ ID 17 and is referred to as KTXHIS-PelbLC-PelbHC in the present invention. Moreover, the plasmid map of KTXHIS-PelbLC-PelbHC is illustrated in figure 3.

The nucleotide sequence of SEQ ID 17 is disclosed below and the sequence of the nucleic acid encoding the light chain fused to PelB signal sequence is underlined and the sequence of the nucleic acid encoding the heavy chain fused to PelB signal sequence is in bold (see also figure 3 for the arrangement of PelB with respect to light and heavy chains of ranibizumab):

ATCTTTCTGCGAATTGAGATGACGCCACTGGCTGGGCGTCATCCCGGTTTCCCGG
GTAAACACCACCGAAAAATAGTTACTATCTTCAAAGCCACATTCGGTCGAAATAT
CACTGATTAACAGGCGGCTATGCTGGAGAAGATATTGCGCATGACACACTCTGAC
CTGTCGCAGATATTGATTGATGGTCATTCCAGTCTGCTGGCGAAATTGCTGACGC
AAAACGCGCTCACTGCACGATGCCTCATCACAAAATTTATCCAGCGCAAAGGGAC
TTTTCAGGCTAGCCGCCAGCCGGGTAATCAGCTTATCCAGCAACGTTTCGCTGGA
TGTTGGCGGCAACGAATCACTGGTGTAACGATGGCGATTCAGCAACATCACCAAC
TGCCCGAACAGCAACTCAGCCATTTCGTTAGCAAACGGCACATGCTGACTACTTT
CATGCTCAAGCTGACCAATAACCTGCCGCGCCTGCGCCATCCCCATGCTACCTAA
GCGCCAGTGTGGTTGCCCTGCGCTGGCGTTAAATCCCGGAATCGCCCCCTGCCAG
TCAAGATTCAGCTTCAGACGCTCCGGGCAATAAATAATATTCTGCAAAACCAGAT
CGTTAACGGAAGCGTAGGAGTGTTTATCATCAGCATGAATGTAAAAGAGATCGCC
ACGGGTAATGCGATAAGGGCGATCGTTGAGTACATGCAGGCCATTACCGCGCCA
GACAATCACCAGCTCACAAAAATCATGTGTATGTTCAGCAAAGACATCTTGCGGA
TAACGGTCAGCCACAGCGACTGCCTGCTGGTCGCTGGCAAAAAAATCATCTTTGA
GAAGTTTTAACTGATGCGCCACCGTGGCTACCTCGGCCAGAGAACGAAGTTGATT
ATTCGCAATATGGCGTACAAATACGTTGAGAAGATTCGCGTTATTGCAGAAAGCC
ATCCCGTCCCTGGCGAATATCACGCGGTGACCAGTTAAACTCTCGGCGAAAAAGC
GTCGAAAAGTGGTTACTGTCGCTGAATCCACAGCGATAGGCGATGTCAGTAACGC
TGGCCTCGCTGTGGCGTAGCAGATGTCGGGCTTTCATCAGTCGCAGGCGGTTCAG
GTATCGCTGAGGCGTCAGTCCCGTTTGCTGCTTAAGCTGCCGATGTAGCGTACGC
AGTGAAAGAGAAAATTGATCCGCCACGGCATCCCAATTCACCTCATCGGCAAAAT
GGTCCTCCAGCCAGGCCAGAAGCAAGTTGAGACGTGATGCGCTGTTTTCCAGGTT
CTCCTGCAAACTGCTTTTACGCAGCAAGAGCAGTAATTGCATAAACAAGATCTCG
CGACTGGCGGTCGAGGGTAAATCATTTTCCCCTTCCTGCTGTTCCATCTGTGCAAC
CAGCTGTCGCACCTGCTGCAATACGCTGTGGTTAACGCGCCAGTGAGACGGATAC
TGCCCATCCAGCTCTTGTGGCAGCAACTGATTCAGCCCGGCGAGAAACTGAAATC
GATCCGGCGAGCGATACAGCACATTGGTCAGACACAGATTATCGGTATGTTCATA
CAGATGCCGATCATGATCGCGTACGAAACAGACCGTGCCACCGGTGATGGTATA

GGGCTGCCCATTAAACACATGAATACCCGTGCCATGTTCGACAATCACAATTTCA
TGAAAATCATGATGATGTTCAGGAAAATCCGCCTGCGGGAGCCGGGGTTCTATCG
CCACGGACGCGTTACCAGACGGAAAAAAATCCACACTATGTAATACGGTCATACT
GGCCTCCTGATGTCGTCAACACGGCGAAATAGTAATCACGAGGTCAGGTTCTTAC
CTTAAATTTTCGACGGAAAACCACGTAAAAAACGTCGATTTTTCAAGATACAGCG
TGAATTTTCAGGAAATGCGGTGAGCATCACATCACCACAATTCAGCAAATTGTGA
ACATCATCACGTTCATCTTTCCCTGGTTGCCAATGGCCCATTTTCTTGTCAGTAAC
GAGAAGGTCGCGAATCCAGGCGCTTTTTAGACTGGTCGTAATGAAATTCAGGAGG
AATTCCTAATGAAATATCTGCTGCCGACCGCAGCAGCGGGTCTGCTGCTGCTGGC
AGCACAGCCTGCAATGGCAGATATTCAGCTGACCCAGAGCCCGAGCAGCCTGAG
CGCAAGCGTTGGTGATCGTGTTACCATTACCTGTAGCGCAAGCCAGGATATTAGC
AATTATCTGAATTGGTATCAGCAGAAACCGGGTAAAGCACCGAAAGTGCTGATCT
ATTTTACCAGCAGCCTGCATAGCGGTGTTCCGAGCCGTTTTAGCGGTAGCGGTAG
TGGCACCGATTTTACCCTGACCATTAGCAGCCTGCAGCCGGAAGATTTTGCAACC
TATTATTGTCAGCAGTATAGCACCGTTCCGTGGACCTTTGGTCAGGGCACCAAAG
TTGAAATTAAACGTACCGTTGCAGCACCGAGCGTTTTTATCTTTCCGCCTAGTGAT
GAACAGCTGAAAAGCGGCACCGCAAGCGTTGTTTGTCTGCTGAATAACTTTTATC
CGCGTGAAGCAAAAGTTCAGTGGAAAGTTGATAATGCACTGCAGAGCGGTAATA
GCCAAGAAAGCGTTACCGAACAGGATAGCAAAGATAGCACCTATAGCCTGAGCA
GCACCCTGACCCTGAGCAAAGCAGATTATGAAAAACACAAAGTGTATGCCTGCG
AAGTTACCCATCAGGGTCTGAGCAGTCCGGTTACCAAAAGTTTTAATCGTGGTGA
ATGCTAATAATCTAGAACTGTTATCGATATCAGGAGGATTAGCATATGAAATATC
TGCTGCCGACCGCAGCAGCGGGTCTGCTGCTGCTGGCAGCACAGCCTGCAA
TGGCAGAAGTTCAGCTGGTTGAAAGCGGTGGTGGTCTGGTTCAGCCTGGTG
GTAGCCTGCGTCTGAGCTGTGCAGCAAGCGGTTATGATTTTACCCATTATGG
TATGAATTGGGTTCGTCAGGCACCGGGTAAAGGTCTGGAATGGGTTGGTTG
GATTAATACCTATACCGGTGAACCGACCTATGCAGCAGATTTTAAACGTCGT
TTTACCTTTAGCCTGGATACCAGCAAAAGCACCGCATATCTGCAGATGAATA
GCCTGCGTGCAGAGGATACCGCAGTGTATTATTGTGCAAAATATCCGTATTA
TTACGGCACCAGCCATTGGTATTTCGATGTTTGGGGTCAGGGCACCCTGGTT
ACCGTTAGCAGCGCAAGCACCAAAGGTCCGAGCGTTTTTCCGCTGGCACCG
AGCAGCAAAAGTACCAGCGGTGGCACCGCAGCACTGGGTTGTCTGGTTAAA
GATTATTTTCCGGAACCGGTTACCGTGAGCTGGAATAGCGGTGCACTGACCA
GCGGTGTTCATACCTTTCCGGCAGTTCTGCAGAGCAGCGGTCTGTATAGCCT

GAGCAGCGTTGTTACCGTTCCGAGCAGCAGCCTGGGCACCCAGACCTATATT
TGTAATGTTAATCATAAACCGAGCAATACCAAAGTGGATAAAAAAGTGGAAC
CGAAAAGCTGCGATAAAACCCATCTGTAATAAAAGCTTGGCTGTTTTGGCGGA
TGAGAGAAGATTTTCAGCCTGATACAGATTAAATCAGAACGCAGAAGCGGTCTG
ATAAAACAGAATTTGCCTGGCGGCAGTAGCGCGGTGGTCCCACCTGACCCCATGC
CGAACTCAGAAGTGAAACGCCGTAGCGCCGATGGTAGTGTGGGGTCTCCCCATGC
GAGAGTAGGGAACTGCCAGGCATCAAATAAAACGAAAGGCTCAGTCGAAAGACT
GGGCCTTTCGTTTTATCTGTTGTTTGTCGGTGAACGCTCTCCTGAGTAGGACAAAT
CCGCCGGGAGCGGATTTGAACGTTGCGAAGCAACGGCCCGGAGGGTGGCGGGCA
GGACGCCCGCCATAAACTGCCAGGCATCAAATTAAGCAGAAGGCCATCCTGACG
GATGGCCTTTTTGCGTTTCTACAAACTCTTTTGTTTATTTTTCTAAATACATTCAAA
TATGTATCCGCTCATGAGACTAGGCTTCCGCGCCCTCATCCGAAAGGGCGTATTC
ATATATGCGGTGTGAAATACCGCACAGATGCGTAAGGAGAAAATACCGCATCAG
GCGCTCTTCCGCTTCCTCGCTCACTGACTCGCTGCGCTCGGTCGTTCGGCTGCGGC
GAGCGGTATCAGCTCACTCAAAGGCGGTAATACGGTTATCCACAGAATCAGGGG
ATAACGCAGGAAAGAACATGTGAGCAAAAGGCCAGCAAAAGGCCAGGAACCGT
AAAAAGGCCGCGTTGCTGGCGTTTTTCCATAGGCTCCGCCCCCCTGACGAGCATC
ACAAAAATCGACGCTCAAGTCAGAGGTGGCGAAACCCGACAGGACTATAAAGAT
ACCAGGCGTTTCCCCCTGGAAGCTCCCTCGTGCGCTCTCCTGTTCCGACCCTGCCG
CTTACCGGATACCTGTCCGCCTTTCTCCCTTCGGGAAGCGTGGCGCTTTCTCATAG
CTCACGCTGTAGGTATCTCAGTTCGGTGTAGGTCGTTCGCTCCAAGCTGGGCTGTG
TGCACGAACCCCCCGTTCAGCCCGACCGCTGCGCCTTATCCGGTAACTATCGTCTT
GAGTCCAACCCGGTAAGACACGACTTATCGCCACTGGCAGCAGCCACTGGTAAC
AGGATTAGCAGAGCGAGGTATGTAGGCGGTGCTACAGAGTTCTTGAAGTGGTGG
CCTAACTACGGCTACACTAGAAGGACAGTATTTGGTATCTGCGCTCTGCTGAAGC
CAGTTACCTTCGGAAAAAGAGTTGGTAGCTCTTGATCCGGCAAACAAACCACCGC
TGGTAGCGGTGGTTTTTTTGTTTGCAAGCAGCAGATTACGCGCAGAAAAAAAGGA
TCTCAAGAAGATCCTTTGATCTTTTCTACGGGGTCTGACGCTCAGTGGAACGAAA
ACTCACGTTAAGGGATTTTGGTCATGAGATTATCAAAAAGGATCTTCACCTAGAT
CCTTTTAAATTAAAAATGAAGTTTTAAATCAATCTAAAGTATATATGAGTAAACT
TGGTCTGACAGTTAGAAAAACTCATCGAGCATCAAATGAAACTGCAATTTATTCA
TATCAGGATTATCAATACCATATTTTTGAAAAAGCCGTTTCTGTAATGAAGGAGA
AAACTCACCGAGGCAGTTCCATAGGATGGCAAGATCCTGGTATCGGTCTGCGATT
CCGACTCGTCCAACATCAATACAACCTATTAATTTCCCCTCGTCAAAAATAAGGT

TATCAAGTGAGAAATCACCATGAGTGACGACTGAATCCGGTGAGAATGGCAAAA
GTTTATGCATTTCTTTCCAGACTTGTTCAACAGGCCAGCCATTACGCTCGTCATCA
AAATCACTCGCATCAACCAAACCGTTATTCATTCGTGATTGCGCCTGAGCGAGAC
GAAATACGCGATCGCTGTTAAAAGGACAATTACAAACAGGAATCGAATGCAACC
GGCGCAGGAACACTGCCAGCGCATCAACAATATTTTCACCTGAATCAGGATATTC
TTCTAATACCTGGAATGCTGTTTTCCCGGGGATCGCAGTGGTGAGTAACCATGCA
TCATCAGGAGTACGGATAAAATGCTTGATGGTCGGAAGAGGCATAAATTCCGTCA
GCCAGTTTAGTCTGACCATCTCATCTGTAACATCATTGGCAACGCTACCTTTGCCA
TGTTTCAGAAACAACTCTGGCGCATCGGGCTTCCCATACAATCGATAGATTGTCG
CACCTGATTGCCCGACATTATCGCGAGCCCATTTATACCCATATAAATCAGCATC
CATGTTGGAATTTAATCGCGGCCTAGAGCAAGACGTTTCCCGTTGAATATGGCTC
ATACTCTTCCTTTTTCAATATTATTGAAGCATTTATCAGGGTTATTGTCTCATGAG
CGGATACATATTTGAATGTATTTAGAAAAATAAACAAATAGGGGTTCCGCGCACA
TTTCCCCGAAAAGTGCCACCTGACGTCTAAGAAACCATTATTATCATGACATTAA
CCTATAAAAATAGGCGTATCACGAGGCCCTTTCGTCT

### Example 3 - Recombinant protein - ranibizumab

The amino acid sequence of the light chain of ranibizumab expressed by the expression vector KTXHIS-PelbLC-PelbHC comprises the sequence of SEQ ID 3:

DIQLTQSPSSLSASVGDRVTITCSASQDISNYLNWYQQKPGKAPKVLIYFTSSLHSGVP
SRFSGSGSGTDFTLTISSLQPEDFATYYCQQYSTVPWTFGQGTKVEIKRTVAAPSVFIF
PPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC

The amino acid sequence of the heavy chain of ranibizumab expressed by the expression vector KTXHIS-PelbLC-PelbHC comprises the sequence of SEQ ID 4:

EVQLVESGGGLVQPGGSLRLSCAASGYDFTHYGMNWVRQAPGKGLEWVGWINTYT
GEPTYAADFKRRFTFSLDTSKSTAYLQMNSLRAEDTAVYYCAKYPYYYGTSHWYFD
VWGQGTLVTVSSASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSC
DKTHL

Moreover, the resulting PelB signal peptide comprises an amino acid sequence of SEQ ID 18 [6]: MKYLLPTAAAGLLLLAAQPAMA

### Example 4 - Superior titer of ranibizumab produced according to the present invention compared to prior art titer

3 different batches comprising ranibizumab were produced according to the below described method.

Minimal media was prepared and inoculated with overnight culture of the XdevK host cell comprising the expression vector KTXHIS-PelbLC-PelbHC from a shake flask. Fermentation temperature was set to 30 °C and the cultures were grown for 22 h in batch phase. This phase was followed by a growth phase for 10 h. The temperature was adjusted to 28.5 °C and the growth rate was then decreased by controlling the glucose feed. The culture was induced with 500 µM of rhamnose injected into the fermenter. The glucose feed was also supplemented with rhamnose for continuous induction throughout the induction phase. Material was harvested after 36 h of induction. The total fermentation time was 68 h and harvested volume was 8530 ml.

After harvest, all material, media and cells, were homogenized. 5500 ml were stored in -80°C and 3000 ml were clarified and washed using TFF. The cells were washed with a volume three times the initial volume for increased release of the product. The resulting product comprising ranibizumab was purified by using capto L affinity chromatography which is also the chromatography method used in Kumar *et al. [4]* (see sections 2.4 and 3.2 in Kumar *et al.*) and then the concentration of purified ranibizumab was determined.

The first, second and third batches comprised a ranibizumab titer of 531 mg/L, 487 mg/L and 518 mg/L, respectively.

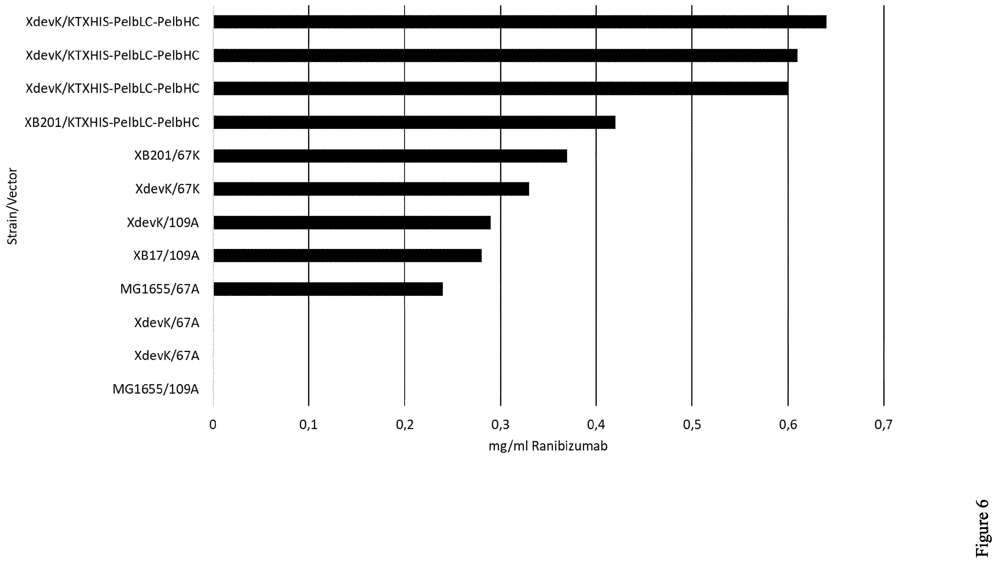

As a comparison, in prior art document Kumar *et al* (which discloses similar purification methods), the ranibizumab titer ranges from 5 mg/L to 25 mg/L as indicated in figure 6 and the Abstract of this document [4].

Consequently, the method according to the present invention (as well as the use of XdevK host cell and expression vector KTXHIS-PelbLC-PelbHC therein) provides unexpectedly higher levels of ranibizumab titer when compared to prior art methods.

### Example 5 - Cell banking

The XdevK was transformed with KTXHIS-PelbLC-PelbHC. A single colony was picked and grown in Defined Bioreactor Medium (DBM) overnight, the following day 20% glycerol was added, the culture was aliquoted and stored in -80°C. One aliquot (50 µl) was used to inoculate 100 ml of Defined Bioreactor Medium (DBM) and cultured for 24h in 30°C, temperature was shifted to 35 °C and the cells were cultured for an additional 6h to increase OD. OD A600nm = 4,44. 20% of glycerol was added, culture was aliquoted (100 µl aliquots) and frozen in -80 °C as RCB100.

Aliquots of the strain were sent for phage testing and microbial purity testing at Charles River Laboratories. The report stated: "It was concluded that RCB100 is free of bacteriophage when tested for both lysogenic prophage and free bacteriophage. The limit of the number of free bacteriophage is less than 1 plaque forming unit per mL of an overnight culture."

### Example 6 - Comparative experiments showing superior titratability

### Materials and methods

The following three strains were used in the comparative experiments:
1. XdevK host cell described in above Example 1; in other words, it is a strain which:
   - has a W3110 strain background having a genotype of: F-, λ-, IN(rrnD-rrnE)1, rph-1);
   - is a K-strain derivative and contains a mutation in the RhaB gene, making it unable to degrade Rhamnose and to use Rhamnose as a carbon source.
2. XB210 described in Hjelm *et al.* [2] which:
   - is a BL21 (DE3) strain having a genotype: F- dompt hsdSB (rB-, mB-) gal dcm (DE3));
   - is a B-strain derivative and contains a mutation in the RhaB gene and a deletion of the Rhamnose transporter gene (RhaT), making it unable to actively transport, degrade Rhamnose and to use Rhamnose as a carbon source;
   - contains parts of the DE3 prophage inserted into the chromosome and does not express the lon protease or the OmpT protease; and
3. MG1655 described in Giacalone *et al.* [5] which is an *E. coli* wild type K- strain.

The following four expression plasmids were used to express the nucleic acid sequence for ranibizumab, i.e. sequences encoding the heavy and light chains of ranibizumab were cloned into the following expression plasmids:
1. KTXHIS;
2. pRha109A described in Giacalone *et al.* [5];
3. pRha67A described in Giacalone *et al.* [5]; and
4. pRha67K described in Hjelm *et al*. [2].

The resulting expression vectors were confirmed by full sequencing.

Moreover, the resulting expression vectors were introduced into the XdevK, XB201 and MG1655 strains (i.e. host cells) to test the different combinations of strains and plasmids to understand the influence on titratability, titer, quality and control of expression.

### Experimental setup for titratability comparisons

The expression vectors comprising the heavy and light chains of ranibizumab were transformed into XdevK, XB201 and MG1655 using standard protocols. Overnight cultures were prepared by inoculating a single colony in 3 ml of LB-veg liquid media containing 100 µg/ml ampicillin or 50 µg/ml kanamycin depending on the construct. Cultures were incubated in a 15 ml falcon tube at 37°C with shaking for 16 hours. Cultures were then back-diluted (1:50) into 5 ml of LB-veg plus antibiotics in a 24-well growth plate, and incubated as before until an OD600 of approximately 0.3-0.5 was reached. Expression was induced by addition of 5, 50, 100, 250, 500, 5000 µM rhamnose and incubation for 5 hours at 30 °C with shaking followed. A volume of cells corresponding to an OD600 of 0,2 units was collected, resuspended in 20 µl 2x Laemlli loading buffer, boiled for 5 mins after which 7.5 µl was analyzed by 12% mini SDS-PAGE. The gel was then immediately transferred to a nitrocellulose/PVDF membrane and probed using anti-Fab antibody for Western blot analysis.

### Results and discussion - titratability

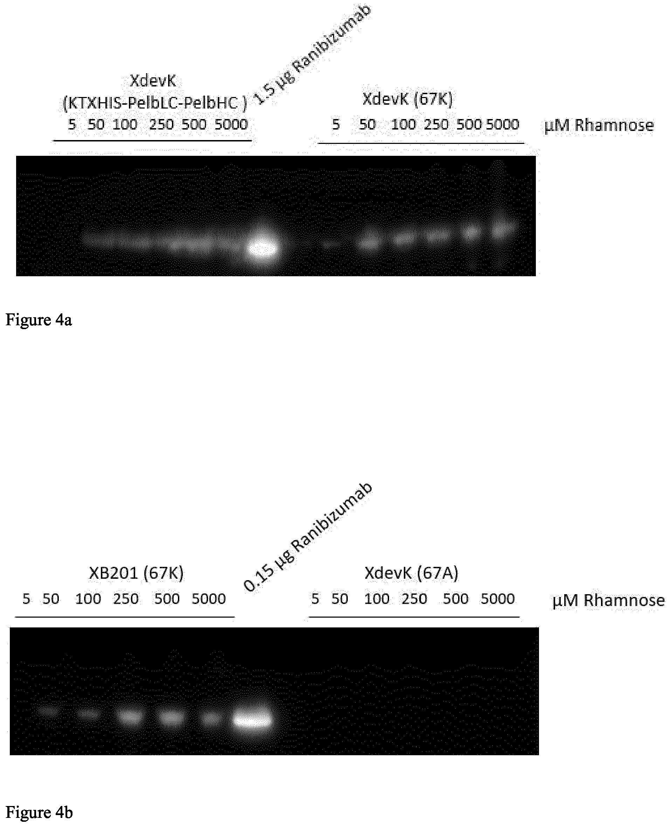

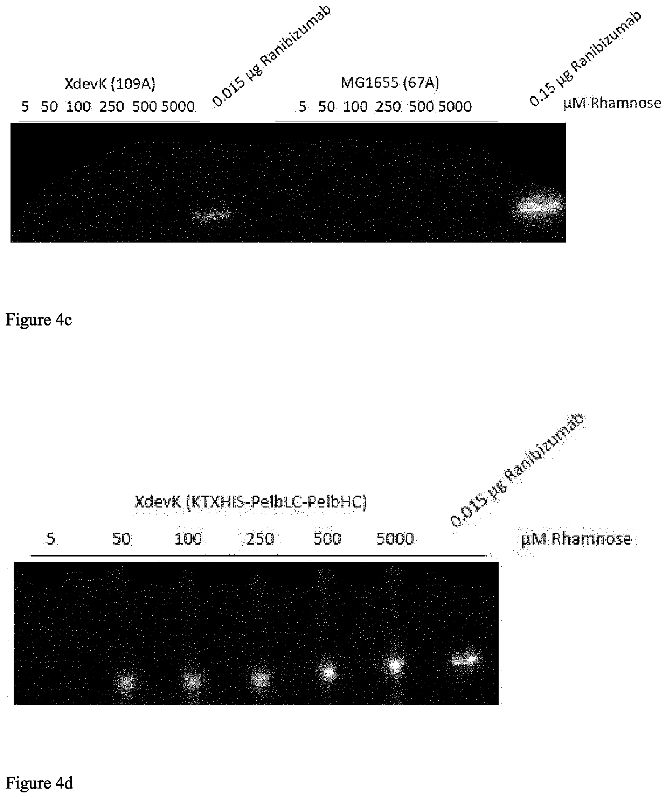

Figures 4a, 4b, 4c and 4d show the Western blots for the titratability comparisons. Each of the control lanes is loaded with purified ranibizumab.

Figure 4a shows:
- the accumulation of expressed ranibizumab in strain XdevK in combination with KTXHIS-PelbLC-PelbHC; and
- the accumulation of expressed ranibizumab in strain XdevK in combination with the expression vector based on the pRha67K (67K) plasmid.

Figure 4b shows:
- the accumulation of expressed ranibizumab in strain XB201 in combination with the expression vector based on the pRha67K (67K) plasmid; and
- the accumulation of expressed ranibizumab in strain XdevK in combination with the expression vector based on the pRha67A (67A) plasmid.

Figure 4c shows:
- the accumulation of expressed ranibizumab in strain XdevK in combination with the expression vector based on the pRha109A (109A) plasmid; and
- the accumulation of expressed ranibizumab in strain MG1655 in combination with the expression vector based on the pRha67A (67A) plasmid.

Figure 4d shows:
- repeating of the experiment showing the accumulation of expressed ranibizumab in strain XdevK in combination with KTXHIS-PelbLC-PelbHC in order to visualize the titratability in combined use of XdevK and the KTXHIS-PelbLC-PelbHC setup.

Figures 4a, 4b, 4c and 4d clearly and unambiguously show that the setup with XdevK in combination with KTXHIS-PelbLC-PelbHC has superior technical effect to the other setups when comparing titratability because:
- the expression of ranibizumab increases gradually for XdevK/KTXHIS-PelbLC-PelbHC each addition of 5, 50, 100, 250, 500, 5000 µM rhamnose; and
- the expression of ranibizumab does not increase gradually in any other combinations - see for example the combination of XB201/67K which gives rise to a "shoulder", i.e. gradual increase but then gradual decrease.

Furthermore, figures 4a, 4b, 4c also show that the cells harboring the expression vectors based on the pRha109A or pRha67A plasmids accumulated very little ranibizumab, at least 100-fold less than the other setups using either KTXHIS-PelbLC-PelbHC or the expression vector based on the pRha67K plasmid (wherein the said fold change was observed by comparing amount of positive control loaded).

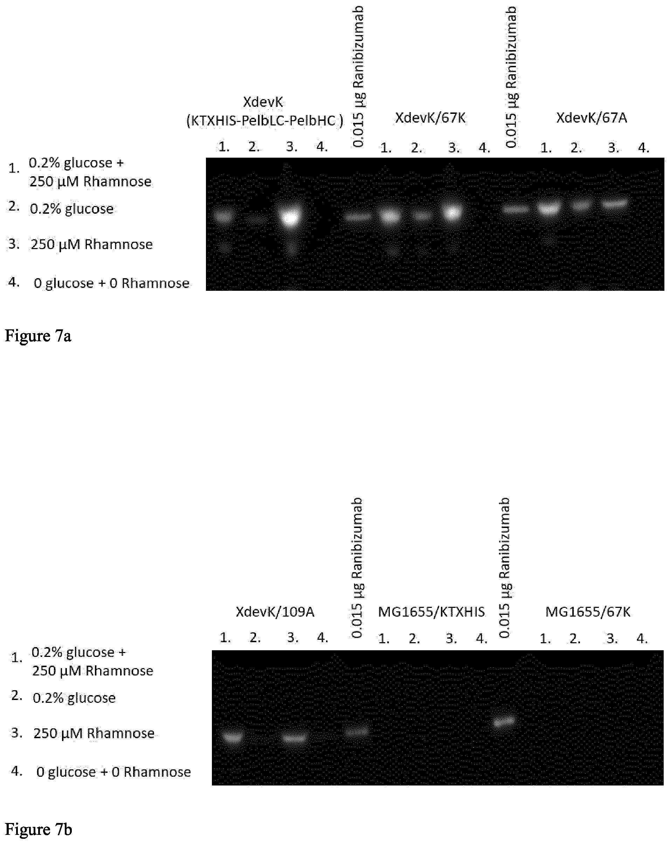

It should be noted that the combinations of:
- MG1655 and the expression vectors based on the pRha109A plasmid (see figure 6),
- MG1655 and the expression vectors based on the pRha67K plasmid (see figure 7b),
- MG1655 and KTXHIS-PelbLC-PelbHC (see figure 7b),
did not give rise to detectable production of ranibizumab and therefore titratability experiments were not conducted for said combinations.

Hence, in conclusion, the XdevK in combination with KTXHIS-PelbLC-PelbHC provides a superior titratability compared to the setups known from prior art. Thus, this allows for a precisely tuning of ranibizumab production rates and therefore the ranibizumab production rates can be set precisely and stably.

### Example 7 - Comparative experiments showing superior quality and titer

### Experimental setup for titer and quality experiments

The expression vectors comprising the heavy and light chains of ranibizumab were transformed into XdevK, XB201 and MG1655 using standard protocols to produce Research Cell Bank (RCB) of respective combination of strain and plasmid. Pre-culture (in shake flask) was inoculated from a single vial of RCB and grown overnight (O/N) in defined reactor media; Potassium dihydrogen phosphate (KH₂PO₄) 4.5 g/L, di-Potassium hydrogen phosphate (K₂HPO₄) 3.0 g/L, Ammonium sulfate ((NH₄)₂SO₄) 3.75 g/L, tri-Sodium citrate dihydrate (HOC(COONa)(CH₂COONa)₂ ·2H₂O) 1.88 g/L, Iron(III) chloride hexahydrate (FeCl₃·6H₂O) 0,0525 g/L, Zinc sulfate heptahydrate (ZnSO₄·7H₂O) 0.0158 g/L, Copper(II) sulfate pentahydrate (CuSO₄·5H₂O) 0.00397 g/L, Manganese sulfate monohydrate (MnSO₄·1H₂O) 0.0198 g/L, Calcium chloride dihydrate (CaCl₂·2H₂O) 0.0207 g/L add 17.1 mL sterile 60% (v/w) glucose solution, 2.432 mL 2.5 M MgSO₄·7H₂O, 1 mL 50 mg/mL Kanamycin solution.

The pH was adjusted by adding 1.5 mL 25% ammonium hydroxide solution (to ca. 6.95). A DAS box fermenter was prepared with defined reactor media and inoculated with O/N pre-culture. The cells growth was controlled by feeding the culture with 60% (v/w) sterile glucose. Production phase was initiated by the addition of rhamnose as the inducer. If necessary, the feed profile was adjusted and salts were added to the culture. The cells were harvested after 20-42 h of induction followed by batch purification. The harvest samples were added to a 96-well deep-well microtiter plate that contained wells with pre-frozen water to guarantee cooling of the sample. Empty wells were filled with MilliQ water (MQ). Sonication was performed using a 24-element probe on a sonics vibra-cell device for 3 cycles of 20 sec each at an amplitude of 40 % with 30 sec pause in-between cycles. The sonicated samples were transferred to 2 mL spin tubes and centrifuged for 20 min at 20 000 x g at 4°C in a centrifuge 5430 R. The supernatant was removed carefully by pipetting and added to a 50 % slurry of the pre-equilibrated Capto L affinity resin. Equilibration had been performed three times with 20 mM sodium phosphate, 150 mM NaCl, pH 7.2 (EQ). 1.9 mL of supernatant was used to load 100 µL of Capto L slurry, which corresponds to a load rate of 9.5 mL/mL resin. The supernatant and the resin were incubated for 15 min at room temperature (RT) while shaking at 1500 rpm on a ThermoMixer C followed by centrifugation for 1 min at 2000 x g at RT. The supernatants were discarded. The resin was washed twice by adding 500 µL of EQ and separating the resin by centrifugation at 2000 x g, as above. During the third wash step using 300 µL of EQ, the resin was transferred to cellulose acetate filter spin cups suitable for 400 µL and centrifuged as above. The fourth wash step was performed with 150 µL EQ. The spin cups were transferred to new collection tubes and elution with 50 mM sodium acetate pH 3.0 was performed in three cycles using 0.4 µL of per µL resin in total (e.g. 3 x 40 µL for 50 µL slurry used). The protein concentration in the eluates was analyzed using an affinity HPLC setup. The quality of the samples was analyzed by SDS-PAGE and SCX-HPLC.

### Results and discussion for titer and quality experiments

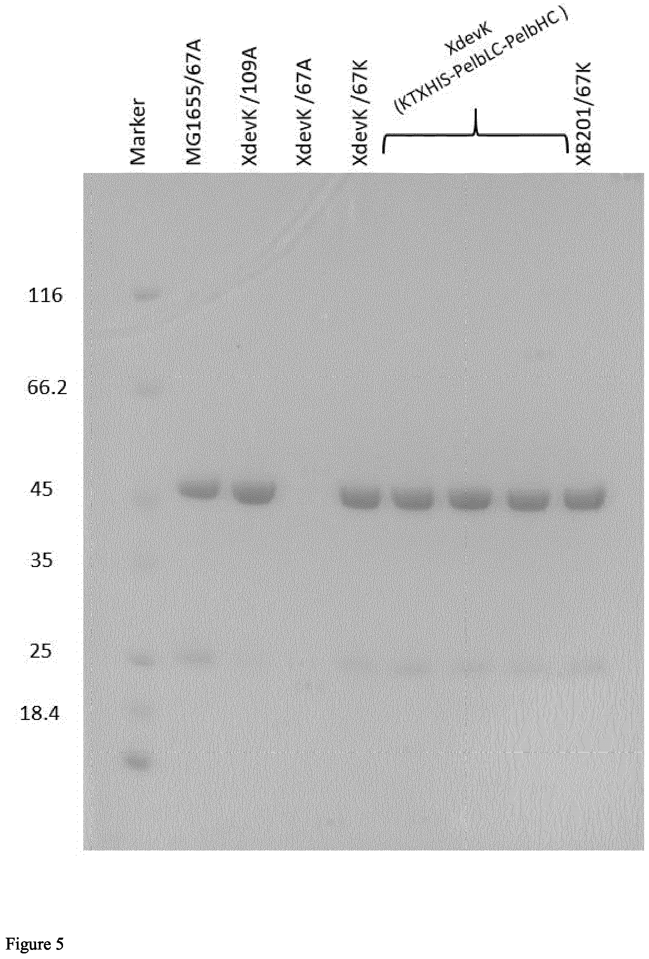

Samples were analyzed using SDS-PAGE as illustrated in figure 5. No detectable quality difference, no increased banding pattern or increased amounts of free light chain or heavy chain, could be detected. However, the setup using the XdevK strain in combination with the expression vector based on the pRha67A plasmid gave very little material and no band could be detected. Furthermore, the setup using strain XdevK in combination with the expression vector based on the pRha109A plasmid grew very poorly and was therefore harvested after a shorter induction time (of 20 hours whereas all other samples were harvested after 42 hours).

The amount of produced material was assessed using affinity HPLC and is summarized in figure 6 which clearly indicates that the setup that produced the highest yield was the XdevK strain in combination with KTXHIS-PelbLC-PelbHC.

Quality of the batch purified material was assessed with SCX-HPLC and is summarized in table 1. The material analyzed from the setup with the XdevK strain in combination with the expression vector based on the pRha67A plasmid is not reliable since this setups produces so little material that it is difficult to analyze. The material from the setup with XdevK strain in combination with the expression vector based on the pRha109A plasmid was harvested after a shorter induction time since the cells grew very slow as compared to the other cells. The SCX HPLC data summarized in table 1 clearly shows that the quality of the material produced in and purified from the XdevK strain in combination with KTXHIS-PelbLC-PelbHC is of superior purity (i.e. quality) as compared to the other setups.

**Table 1. Summary of SCX HPLC purity data.**

| **Description** | **MG1655/67A** | **XdevK /109A** | **XdevK /67A** | **XdevK /67K** | **XdevK / KTXHIS-PelbLC-PelbHC** | **XB201/67A** |
|---|---|---|---|---|---|---|
| Ranibizumab purity in % | 30.6 | 53.0 | 59.3 | 73.8 | 83.0 | 71.5 |

Hence, in conclusion, the XdevK strain in combination with KTXHIS-PelbLC-PelbHC is superior compared to the other setups both in the quality of the produced ranibizumab and the yield of the produced ranibizumab.

### Example 8 - Comparative experiments showing superior control of expression

### Experimental setup for assessing control of expression

The expression vectors comprising the heavy and light chains of ranibizumab were transformed into XdevK and MG1655 using standard protocols. Overnight cultures were prepared by inoculating a single colony in 3 ml of LB-veg liquid media containing 100 µg/ml ampicillin or 50 µg/ml kanamycin depending on the plasmid used. Cultures were incubated in a 15 ml falcon tube at 37°C with shaking for 16 hours. Cultures were then back-diluted (1:50) into 5 ml of LB-veg plus antibiotics in a 24-well growth plate, and incubated as before until an OD600 of approximately 0.3-0.5 was reached and different expression conditions were analyzed. One set of the cultures was grown in the presence of 0.2% glucose. Expression was induced by addition of 250 µM rhamnose in a subset of the cultures and incubated at 30 °C with shaking. A volume of cells corresponding to an OD600 of 0,2 units was collected (spun down) after 24 hours, resuspended in 20 µl 2x Laemlli loading buffer, boiled for 5 mins and analyzed (7,5 µl) by 12% mini SDS-PAGE. The gel was then immediately transferred to a nitrocellulose/PVDF membrane and probed using anti-Fab antibody.

### Results and discussion for control of expression

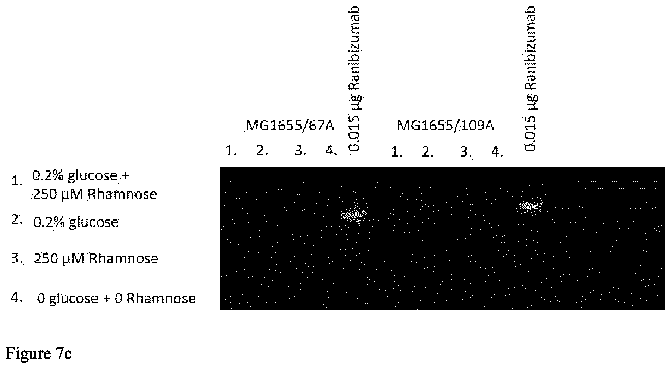

Figures 7a, 7b and 7c show the Western blots for the comparison of expression control. Each of the control lanes is loaded with ranibizumab. Samples in lane 1 from each setup were grown in the presence of 0.2% glucose and induced with 250 µm rhamnose. Samples in lane 2 from each setup were grown in the presence of 0.2% glucose. Samples in lane 3 from each setup were induced with 250 µm rhamnose. Samples in lane 4 from each setup were grown without any additions.

Figure 7a shows:
- the accumulation of expressed ranibizumab in strain XdevK in combination with KTXHIS-PelbLC-PelbHC; and
- the accumulation of expressed ranibizumab in strain XdevK in combination with the expression vector based on the pRha67K (67K) plasmid; and
- the accumulation of expressed ranibizumab in strain XdevK in combination with the expression vector based on the pRha67A (67A) plasmid.

Figure 7b shows:
- the accumulation of expressed ranibizumab in strain XdevK in combination with the expression vector based on the pRha109A (109A) plasmid;
- the accumulation of expressed ranibizumab in strain MG1655 in combination with KTXHIS-PelbLC-PelbHC;
- the accumulation of expressed ranibizumab in strain MG1655 in combination with the expression vector based on the pRha67K (67K) plasmid.

Figure 7c shows:
- the accumulation of expressed ranibizumab in strain MG1655 in combination with the expression vector based on the pRha67A (67A) plasmid; and
- the accumulation of expressed ranibizumab in strain MG1655 in combination with the expression vector based on the pRha109A (109A) plasmid.

When the accumulation of ranibizumab is compared between the different setup in figures 7a-7c, it is clear that highest expression of ranibizumab, induced by the addition of 250 µM rhamnose as indicated in lanes 3, was detected in XdevK strain in combination with KTXHIS-PelbLC-PelbHC.

Interestingly, as indicated in lanes 3 in figures 7b and 7c, none of the setups involving the MG1655 strain resulted in visible expression of ranibizumab.

Moreover, of the setups that resulted in the expression of ranibizumab in lanes 3 (i.e. the setups in which the XdevK strain was used), the XdevK strain in combination with KTXHIS-PelbLC-PelbHC resulted in the lowest expression of ranibizumab when grown in the presence of 0.2% glucose as indicated in lanes 2. This suggest that the XdevK strain in combination with KTXHIS-PelbLC-PelbHC is less prone to leaky expression in the absence of inducer. Leaky expression can have a negative effect on growth of the cells leading to lower overall yield. Leaky expression can also affect the quality of the produced material leading to a mixture of different species of the accumulated expressed protein that can be problematic to handle in a downstream purification step and therefore lead to a lower yield and a complicate purification process

Hence, the control of ranibizumab expression in the XdevK strain in combination with KTXHIS-PelbLC-PelbHC is superior when compared to the other setups.

In conclusion, the experimental results shown in Examples 4, 6, 7 and 8 demonstrate that the present invention provides the following advantageous and unexpected effects when compared with prior art expression vectors and/or host cells and therefore in particular is suitable for the industrial production of ranibizumab
- superior titratability (example 6);
- superior purity and quality (example 7);
- superior titer (examples 4, 7 and 8); and
- superior control of expression (example 8).

### REFERENCES

1. Holcroft CC et al. Interdependence of activation at rhaSR by cyclic AMP receptor protein, the RNA polymerase alpha subunit C-terminal domain, and rhaR. J Bacteriol. 2000;182(23): 6774-6782
2. Hjelm, A. et al. Tailoring Escherichia coli for the l-Rhamnose PBAD Promoter-Based Production of Membrane and Secretory Proteins. ACS synthetic biology 2017; 6 6: 985-994 .
3. Wilms, B. et al. High-cell-density fermentation for production of L-N-carbamoylase using an expression system based on the Escherichia coli rhaBAD promoter. Biotechnol. Bioeng. 2001; 73: 95-103
4. Kumar, D. et al. QbD Based Media Development for the Production of Fab Fragments in E. coli. Bioengineering 2019; 6, 29.
5. Giacalone MJ et al. "Toxic protein expression in Escherichia coli using a rhamnose-based tightly regulated and tunable promoter system. Biotechniques. 2006; 40(3): 355-364.
6. Choi, J.H. et al. Secretory and extracellular production of recombinant proteins using Escherichia coli. Appl Microbiol Biotechnol 2004; 64, 625-635
7. Sandomenico A. et al. Evolution of Escherichia coli Expression System in Producing Antibody Recombinant Fragments. Int. J. Mol. Sci. 2020: 21:6324

## Claims

1. DNA construct for expressing ranibizumab in a bacterial host cell, wherein ranibizumab comprises (i) a light chain comprising the amino acid sequence of SEQ ID 3, and (ii) a heavy chain comprising the amino acid sequence of SEQ ID 4, wherein said DNA construct comprises a nucleotide sequence encoding ranibizumab, wherein said DNA construct further comprises at least one nucleotide sequence encoding a signal peptide which is operably linked in the direction of transcription to the nucleotide sequence encoding the light chain of ranibizumab and/or the heavy chain of ranibizumab,
wherein said DNA construct further comprises nucleotide sequences encoding:
- *rhaBAD* promoter,
- RhaR transcription activator,
- RhaS transcription activator,
- an antibiotic resistance marker,
- a promoter operably linked to the nucleotide sequence encoding the antibiotic resistance marker
- rrnB T1 terminator
- rrnB T2 terminator, and
- a pMB1 origin of replication.

2. DNA construct according to claim 1, wherein
- the antibiotic resistance marker is a kanamycin resistance marker, preferably a kanamycin resistance marker comprising the nucleotide sequence of SEQ ID 12 or a sequence with at least 90 % sequence identity thereto;
- the promoter operably linked to the nucleotide sequence encoding the antibiotic resistance marker is an AmpR promoter, preferably an AmpR promoter comprising the nucleotide sequence of SEQ ID 13 or a sequence with at least 90 % sequence identity thereto;
- the rrnB T1 terminator comprises a nucleotide sequence of SEQ ID 14 or a sequence with at least 90 % sequence identity thereto;
- the rrnB T2 terminator comprises the nucleotide sequence of SEQ ID 15 or a sequence with at least 90 % sequence identity thereto; and/or
- the pMB1 origin of replication comprises the nucleotide sequence of SEQ ID 16 or a sequence with at least 90 % sequence identity thereto.

3. DNA construct according to claim 1 or 2, wherein
- the antibiotic resistance marker is a kanamycin resistance marker comprising the nucleotide sequence of SEQ ID 12;
- the promoter operably linked to the nucleotide sequence encoding the antibiotic resistance marker is an AmpR promoter comprising the nucleotide sequence of SEQ ID 13;
- the rrnB T1 terminator comprises the nucleotide sequence of SEQ ID 14;
- the rrnB T2 terminator comprises the nucleotide sequence of SEQ ID 15; and/or
- the pMB1 origin of replication comprises the nucleotide sequence of SEQ ID 16.

4. DNA construct according to claim 1 or 2, wherein:
- the *rhaBAD* promoter comprises the nucleotide sequence of SEQ ID 8 or a sequence with at least 90 % sequence identity thereto, preferably the nucleotide sequence of SEQ ID 8;
- the RhaR transcription activator comprises the nucleotide sequence of SEQ ID 9 or a sequence with at least 90 % sequence identity thereto, preferably the nucleotide sequence of SEQ ID 9;
- the RhaS transcription activator comprises the nucleotide sequence of SEQ ID 11 or a sequence with at least 90 % sequence identity thereto, preferably the nucleotide sequence of SEQ ID 11;
- the antibiotic resistance marker is a kanamycin resistance marker comprising the nucleotide sequence of SEQ ID 12 or a sequence with at least 90 % sequence identity thereto, preferably the nucleotide sequence of SEQ ID 12;
- the AmpR promoter comprises the nucleotide sequence of SEQ ID 13 or a sequence with at least 90 % sequence identity thereto, preferably the nucleotide sequence of SEQ ID 13;
- the rrnB T1 terminator comprises the nucleotide sequence of SEQ ID 14 or a sequence with at least 90 % sequence identity thereto, preferably the nucleotide sequence of SEQ ID 14;
- the rrnB T2 terminator comprises the nucleotide sequence of SEQ ID 15 or a sequence with at least 90 % sequence identity thereto, preferably the nucleotide sequence of SEQ ID 15; and
- the pMB 1 origin of replication comprises the nucleotide sequence of SEQ ID 16 or a sequence with at least 90 % sequence identity thereto, preferably the nucleotide sequence of SEQ ID 16.

5. DNA construct according to any one of claims 1-4, wherein the nucleotide sequence encoding ranibizumab is operably linked to the *rhaBAD* promoter.

6. DNA construct according to any one of claims 1-5, wherein the nucleotide sequence encoding ranibizumab comprises (i) a nucleotide sequence encoding the light chain of ranibizumab comprising the sequence of SEQ ID 5 or a sequence with at least 90 % sequence identity thereto, and/or (ii) a nucleotide sequence encoding for the heavy chain of ranibizumab comprising the sequence of SEQ ID 6 or a sequence with at least 90 % sequence identity thereto.

7. DNA construct according to any one of claims 1-6, wherein the nucleotide sequence encoding ranibizumab comprises (i) a nucleotide sequence encoding for the light chain of ranibizumab comprising the sequence of SEQ ID 5, and/or (ii) a nucleotide sequence encoding for the heavy chain of ranibizumab comprising the sequence of SEQ ID 6.

8. DNA construct according to claim 7, wherein the nucleotide sequence encoding a signal peptide is operably linked in the direction of transcription to either one or both of nucleotide sequences of SEQ ID 5 and SEQ ID 6.

9. DNA construct according to any one of claims 1-8, wherein the signal peptide is PelB.

10. DNA construct according to claim 9, wherein the nucleotide sequence encoding the PelB signal peptide comprises the sequence of SEQ ID 7 or a sequence with at least 90 % sequence identity thereto, preferably the nucleotide sequence encoding the PelB signal peptide comprises the sequence of SEQ ID 7.

11. DNA construct according to claim 9 or 10, wherein said DNA construct comprises the sequence of SEQ ID 17 or a sequence with at least 90 % sequence identity thereto, preferably comprises a sequence of SEQ ID 17.

12. Expression vector comprising the DNA construct according to any one of claims 1-11.

13. Bacterial host cell comprising the DNA construct according to any of claims 1-11 or the expression vector according to claim 12, wherein said bacterial host cell is preferably an *Escherichia coli* cell, more preferably an *E. coli* K-12 cell.

14. Host cell according to claim 13, wherein said host cell either comprises (i) a chromosome which comprises a mutation in the nucleotide sequence of the *rhaB* gene which renders RhaB inactive, or (ii) a chromosome in which the nucleotide sequence encoding RhaB is deleted.

15. Host cell according to claim 13 or 14, wherein said host cell is an *Escherichia coli* W3110 cell, preferably comprising a chromosome which comprises a frame shift-mutation in the nucleotide sequence encoding RhaB.

16. Host cell according to any one of claims 13-15, wherein said host cell is an *Escherichia coli* W3110 cell comprising a chromosome which comprises the nucleotide sequence of SEQ ID 2 or a sequence with at least 90 % sequence identity thereto, preferably the nucleotide sequence of SEQ ID 2; wherein optionally, the chromosome of the host cell further comprises a nucleotide sequence encoding RhaT.

17. Method of producing ranibizumab comprising the step of exposing the bacterial host cell according to any of claims 13-16 to rhamnose, thereby inducing expression of ranibizumab.

18. Method according to claim 17, further comprising the step of recovering ranibizumab from the bacterial host cell; optionally further comprising one or more step(s) of purifying the recovered ranibizumab, preferably by one or more chromatography steps.

## Patentansprüche

1. DNA-Konstrukt zur Expression von Ranibizumab in einer bakteriellen Wirtszelle, wobei Ranibizumab (i) eine leichte Kette, umfassend die Aminosäuresequenz von SEQ ID 3, und (ii) eine schwere Kette, umfassend die Aminosäuresequenz von SEQ ID 4, umfasst, wobei das DNA-Konstrukt eine Nukleotidsequenz umfasst, die für Ranibizumab kodiert, wobei das DNA-Konstrukt weiter mindestens eine Nukleotidsequenz umfasst, die für ein Signalpeptid kodiert, das in Transkriptionsrichtung mit der Nukleotidsequenz, die für die leichte Kette von Ranibizumab und/oder die schwere Kette von Ranibizumab kodiert, funktionell verbunden ist,
wobei das DNA-Konstrukt weiter Nukleotidsequenzen umfasst, die für folgendes kodieren:
- *rhaBAD*-Promotor,
- RhaR-Transkriptionsaktivator,
- RhaS-Transkriptionsaktivator,
- einen Antibiotikaresistenzmarker,
- einen Promotor, der funktionell mit der Nukleotidsequenz verbunden ist, die für den Antibiotikaresistenzmarker kodiert,
- rrnB T1-Terminator,
- rrnB T2-Terminator , und
- einen pMB1-Replikationsursprung.

2. DNA-Konstrukt nach Anspruch 1, wobei
- der Antibiotikaresistenzmarker ein Kanamycin-Resistenzmarker ist, bevorzugt ein Kanamycin-Resistenzmarker, der die Nukleotidsequenz von SEQ ID 12 oder eine Sequenz mit mindestens 90 % Sequenzidentität dazu umfasst;
- der Promotor, der funktionell mit der Nukleotidsequenz verbunden ist, die für den Antibiotikaresistenzmarker kodiert, ein AmpR-Promotor ist, bevorzugt ein AmpR-Promotor, der die Nukleotidsequenz von SEQ ID 13 oder eine Sequenz mit mindestens 90 % Sequenzidentität dazu umfasst;
- der rrnB T1-Terminator die Nukleotidsequenz von SEQ ID 14 oder eine Sequenz mit mindestens 90 % Sequenzidentität dazu umfasst;
- der rrnB T2-Terminator die Nukleotidsequenz von SEQ ID 15 oder eine Sequenz mit mindestens 90 % Sequenzidentität dazu umfasst; und/oder
- der pMB1-Replikationsursprung die Nukleotidsequenz von SEQ ID 16 oder eine Sequenz mit mindestens 90 % Sequenzidentität dazu umfasst.

3. DNA-Konstrukt nach Anspruch 1 oder 2, wobei
- der Antibiotikaresistenzmarker ein Kanamycin-Resistenzmarker ist, der die Nukleotidsequenz von SEQ ID 12 umfasst;
- der Promotor, der funktionell mit der Nukleotidsequenz verbunden ist, die für den Antibiotikaresistenzmarker kodiert, ein AmpR-Promotor ist, der die Nukleotidsequenz von SEQ ID 13 umfasst;
- der rrnB T1-Terminator die Nukleotidsequenz von SEQ ID 14 umfasst;
- der rrnB T2-Terminator die Nukleotidsequenz von SEQ ID 15 umfasst; und/oder
- der pMB1-Replikationsursprung die Nukleotidsequenz von SEQ ID 16 umfasst.

4. DNA-Konstrukt nach Anspruch 1 oder 2, wobei:
- der *rhaBAD*-Promotor die Nukleotidsequenz von SEQ ID 8 oder eine Sequenz mit mindestens 90 % Sequenzidentität dazu umfasst, bevorzugt die Nukleotidsequenz von SEQ ID 8;
- der RhaR-Transkriptionsaktivator die Nukleotidsequenz von SEQ ID 9 oder eine Sequenz mit mindestens 90 % Sequenzidentität dazu umfasst, bevorzugt die Nukleotidsequenz von SEQ ID 9;
- der RhaS-Transkriptionsaktivator die Nukleotidsequenz von SEQ ID 11 oder eine Sequenz mit mindestens 90 % Sequenzidentität dazu umfasst, bevorzugt die Nukleotidsequenz von SEQ ID 11;
- der Antibiotikaresistenzmarker ein Kanamycin-Resistenzmarker ist, der die Nukleotidsequenz von SEQ ID 12 oder eine Sequenz mit mindestens 90 % Sequenzidentität dazu umfasst, bevorzugt die Nukleotidsequenz von SEQ ID 12;
- der AmpR-Promotor die Nukleotidsequenz von SEQ ID 13 oder eine Sequenz mit mindestens 90 % Sequenzidentität dazu umfasst, bevorzugt die Nukleotidsequenz von SEQ ID 13
- der rrnB T1-Terminator die Nukleotidsequenz von SEQ ID 14 oder eine Sequenz mit mindestens 90 % Sequenzidentität dazu umfasst, bevorzugt die Nukleotidsequenz von SEQ ID 14
- der rrnB T2-Terminator die Nukleotidsequenz von SEQ ID 15 oder eine Sequenz mit mindestens 90 % Sequenzidentität dazu umfasst, bevorzugt die Nukleotidsequenz von SEQ ID 15; und
- der pMB1-Replikationsursprung die Nukleotidsequenz von SEQ ID 16 oder eine Sequenz mit mindestens 90 % Sequenzidentität dazu umfasst, bevorzugt die Nukleotidsequenz von SEQ ID 16.

5. DNA-Konstrukt nach einem der Ansprüche 1-4, wobei die Nukleotidsequenz, die für Ranibizumab kodiert, funktionell mit dem *rhaBAD*-Promotor verbunden ist.

6. DNA-Konstrukt nach einem der Ansprüche 1-5, wobei die Nukleotidsequenz, die für Ranibizumab kodiert, (i) eine Nukleotidsequenz, die für die leichte Kette von Ranibizumab kodiert, umfassend die Sequenz von SEQ ID 5 oder eine Sequenz mit mindestens 90 % Sequenzidentität dazu, und/oder (ii) eine Nukleotidsequenz, die für die schwere Kette von Ranibizumab kodiert, umfassend die Sequenz von SEQ ID 6 oder eine Sequenz mit mindestens 90 % Sequenzidentität dazu, umfasst.

7. DNA-Konstrukt nach einem der Ansprüche 1-6, wobei die Nukleotidsequenz, die für Ranibizumab kodiert, (i) eine Nukleotidsequenz, die für die leichte Kette von Ranibizumab kodiert, umfassend die Sequenz von SEQ ID 5, und/oder (ii) eine Nukleotidsequenz, die für die schwere Kette von Ranibizumab kodiert, umfassend die Sequenz von SEQ ID 6, umfasst.

8. DNA-Konstrukt nach Anspruch 7, wobei die Nukleotidsequenz, die für ein Signalpeptid kodiert, in Transkriptionsrichtung mit entweder einer oder beiden Nukleotidsequenzen von SEQ ID 5 und SEQ ID 6 funktionell verbunden ist.

9. DNA-Konstrukt nach einem der Ansprüche 1-8, wobei das Signalpeptid PelB ist.

10. DNA-Konstrukt nach Anspruch 9, wobei die Nukleotidsequenz, die für das PelB-Signalpeptid kodiert, die Sequenz von SEQ ID 7 oder eine Sequenz mit mindestens 90 % Sequenzidentität dazu umfasst, vorzugsweise umfasst die Nukleotidsequenz, die für das PelB-Signalpeptid kodiert, die Sequenz von SEQ ID 7.

11. DNA-Konstrukt nach Anspruch 9 oder 10, wobei das DNA-Konstrukt die Sequenz von SEQ ID 17 oder eine Sequenz mit mindestens 90 % Sequenzidentität dazu umfasst, bevorzugt die Sequenz von SEQ ID 17 umfasst.

12. Expressionsvektor, der das DNA-Konstrukt nach einem der Ansprüche 1-11 umfasst.

13. Bakterielle Wirtszelle, die das DNA-Konstrukt nach einem der Ansprüche 1 bis 11 oder den Expressionsvektor nach Anspruch 12 umfasst, wobei die bakterielle Wirtszelle bevorzugt eine *Escherichia coli*-Zelle, noch bevorzugter eine *E*. *coli* K-12-Zelle ist.

14. Wirtszelle nach Anspruch 13, wobei die Wirtszelle entweder (i) ein Chromosom, das eine Mutation in der Nukleotidsequenz des *rhaB*-Gens umfasst, die RhaB inaktiviert, oder (ii) ein Chromosom, in dem die Nukleotidsequenz, die für RhaB kodiert, deletiert ist, umfasst.

15. Wirtszelle nach Anspruch 13 oder 14, wobei die Wirtszelle eine *Escherichia coli* W3110-Zelle ist, die bevorzugt ein Chromosom umfasst, das eine
Leserahmenverschiebungsmutation in der für RhaB kodierenden Nukleotidsequenz umfasst.

16. Wirtszelle nach einem der Ansprüche 13 bis 15, wobei die Wirtszelle eine *Escherichia coli* W3110-Zelle ist, die ein Chromosom umfasst, das die Nukleotidsequenz von SEQ ID 2 oder eine Sequenz mit mindestens 90 % Sequenzidentität dazu, bevorzugt die Nukleotidsequenz von SEQ ID 2, umfasst; wobei das Chromosom der Wirtszelle optional weiter eine Nukleotidsequenz umfasst, die für RhaT codiert.

17. Verfahren zur Herstellung von Ranibizumab, umfassend den Schritt des Aussetzens der bakteriellen Wirtszelle nach einem der Ansprüche 13-16 gegenüber Rhamnose, wodurch die Expression von Ranibizumab induziert wird.

18. Verfahren nach Anspruch 17, das weiter den Schritt der Gewinnung von Ranibizumab aus der bakteriellen Wirtszelle umfasst; das optional weiter einen oder mehrere Schritte zur Reinigung des gewonnenen Ranibizumab umfasst, bevorzugt durch einen oder mehrere Chromatographieschritte.

## Revendications

1. Construction d'ADN destinée à exprimer du ranibizumab dans une cellule hôte bactérienne, sachant que le ranibizumab comprend (i) une chaîne légère comprenant la séquence d'acides aminés de SEQ ID 3, et (ii) une chaîne lourde comprenant la séquence d'acides aminés de SEQ ID 4, sachant que ladite construction d'ADN comprend une séquence de nucléotides codant du ranibizumab, sachant que ladite construction d'ADN comprend en outre au moins une séquence de nucléotides codant un peptide signal qui est lié de manière opérationnelle dans la direction de transcription à la séquence de nucléotides codant la chaîne légère de ranibizumab et/ou la chaîne lourde de ranibizumab,
sachant que ladite construction d'ADN comprend en outre des séquences de nucléotides codant :
- un promoteur *rhaBAD*,
- un activateur de transcription RhaR,
- un activateur de transcription RhaS,
- un marqueur de résistance aux antibiotiques,
- un promoteur lié de manière opérationnelle à la séquence de nucléotides codant le marqueur de résistance aux antibiotiques,
- un terminateur rrnB T1,
- un terminateur rrnB T2, et
- une origine de réplication pMB1.

2. Construction d'ADN selon la revendication 1, sachant que
- le marqueur de résistance aux antibiotiques est un marqueur de résistance à la kanamycine, de préférence un marqueur de résistance à la kanamycine comprenant la séquence de nucléotides de SEQ ID 12 ou une séquence ayant au moins 90 % d'identité de séquence avec celle-ci ;
- le promoteur lié de manière opérationnelle à la séquence de nucléotides codant le marqueur de résistance aux antibiotiques est un promoteur AmpR, de préférence un promoteur AmpR comprenant la séquence de nucléotides de SEQ ID 13 ou une séquence ayant au moins 90 % d'identité de séquence avec celle-ci ;
- le terminateur rrnB T1 comprend une séquence de nucléotides de SEQ ID 14 ou une séquence ayant au moins 90 % d'identité de séquence avec celle-ci ;
- le terminateur rrnB T2 comprend la séquence de nucléotides de SEQ ID 15 ou une séquence ayant au moins 90 % d'identité de séquence avec celle-ci ; et/ou
- l'origine de réplication pMB1 comprend la séquence de nucléotides de SEQ ID 16 ou une séquence ayant au moins 90 % d'identité de séquence avec celle-ci.

3. Construction d'ADN selon la revendication 1 ou 2, sachant que
- le marqueur de résistance aux antibiotiques est un marqueur de résistance à la kanamycine comprenant la séquence de nucléotides de SEQ ID 12 ;
- le promoteur lié de manière opérationnelle à la séquence de nucléotides codant le marqueur de résistance aux antibiotiques est un promoteur AmpR comprenant la séquence de nucléotides de SEQ ID 13 ;
- le terminateur rrnB T1 comprend la séquence de nucléotides de SEQ ID 14 ;
- le terminateur rrnB T2 comprend la séquence de nucléotides de SEQ ID 15 ; et/ou
- l'origine de réplication pMB1 comprend la séquence de nucléotides de SEQ ID 16.

4. Construction d'ADN selon la revendication 1 ou 2, sachant que
- le promoteur rhaBAD comprend la séquence de nucléotides de SEQ ID 8 ou une séquence ayant au moins 90 % d'identité de séquence avec celle-ci, de préférence la séquence de nucléotides de SEQ ID 8 ;
- l'activateur de transcription RhaR comprend la séquence de nucléotides de SEQ ID 9 ou une séquence ayant au moins 90 % d'identité de séquence avec celle-ci, de préférence la séquence de nucléotides de SEQ ID 9 ;
- l'activateur de transcription RhaS comprend la séquence de nucléotides de SEQ ID 11 ou une séquence ayant au moins 90 % d'identité de séquence avec celle-ci, de préférence la séquence de nucléotides de SEQ ID 11 ;
- le marqueur de résistance aux antibiotiques est un marqueur de résistance à la kanamycine comprenant la séquence de nucléotides de SEQ ID 12 ou une séquence ayant au moins 90 % d'identité de séquence avec celle-ci, de préférence la séquence de nucléotides de SEQ ID 12 ;
- le promoteur AmpR comprend la séquence de nucléotides de SEQ ID 13 ou une séquence ayant au moins 90 % d'identité de séquence avec celle-ci, de préférence la séquence de nucléotides de SEQ ID 13 ;
- le terminateur rrnB T1 comprend la séquence de nucléotides de SEQ ID 14 ou une séquence ayant au moins 90 % d'identité de séquence avec celle-ci, de préférence la séquence de nucléotides de SEQ ID 14 ;
- le terminateur rrnB T2 comprend la séquence de nucléotides de SEQ ID 15 ou une séquence ayant au moins 90 % d'identité de séquence avec celle-ci, de préférence la séquence de nucléotides de SEQ ID 15 ; et
- l'origine de réplication pMB1 comprend la séquence de nucléotides de SEQ ID 16 ou une séquence ayant au moins 90 % d'identité de séquence avec celle-ci, de préférence la séquence de nucléotides de SEQ ID 16.

5. Construction d'ADN selon l'une quelconque des revendications 1 à 4, sachant que la séquence de nucléotides codant du ranibizumab est liée de manière opérationnelle au promoteur *rhaBAD*.

6. Construction d'ADN selon l'une quelconque des revendications 1 à 5, sachant que la séquence de nucléotides codant du ranibizumab comprend (i) une séquence de nucléotides codant la chaîne légère de ranibizumab comprenant la séquence de SEQ ID 5 ou une séquence ayant au moins 90 % d'identité de séquence avec celle-ci, et/ou (ii) une séquence de nucléotides codant la chaîne lourde de ranibizumab comprenant la séquence de SEQ ID 6 ou une séquence ayant au moins 90 % d'identité de séquence avec celle-ci.

7. Construction d'ADN selon l'une quelconque des revendications 1 à 6, sachant que la séquence de nucléotides codant du ranibizumab comprend (i) une séquence de nucléotides codant la chaîne légère de ranibizumab comprenant la séquence de SEQ ID 5, et/ou (ii) une séquence de nucléotides codant la chaîne lourde de ranibizumab comprenant la séquence de SEQ ID 6.

8. Construction d'ADN selon la revendication 7, sachant que la séquence de nucléotides codant un peptide signal est liée de manière opérationnelle dans la direction de transcription à l'une ou l'autre ou aux deux des séquences de nucléotides de SEQ ID 5 et SEQ ID 6.

9. Construction d'ADN selon l'une quelconque des revendications 1 à 8, sachant que le peptide signal est PelB.

10. Construction d'ADN selon la revendication 9, sachant que la séquence de nucléotides codant le peptide signal PelB comprend la séquence de SEQ ID 7 ou une séquence ayant au moins 90 % d'identité de séquence avec celle-ci, de préférence la séquence de nucléotides codant le peptide signal PelB comprend la séquence de SEQ ID 7.

11. Construction d'ADN selon la revendication 9 ou 10, sachant que ladite construction d'ADN comprend la séquence de SEQ ID 17 ou une séquence ayant au moins 90 % d'identité de séquence avec celle-ci, comprend de préférence une séquence de SEQ ID 17.

12. Vecteur d'expression comprenant la construction d'ADN selon l'une quelconque des revendications 1 à 11.

13. Cellule hôte bactérienne comprenant la construction d'ADN selon l'une quelconque des revendications 1 à 11 ou le vecteur d'expression selon la revendication 12, sachant que ladite cellule hôte bactérienne est de préférence une cellule d'*Escherichia coli,* de manière plus préférentielle une cellule d'*E. coli* K-12.

14. Cellule hôte selon la revendication 13, sachant que ladite cellule hôte comprend soit (i) un chromosome qui comprend une mutation dans la séquence de nucléotides du gène *rhaB* qui rend *RhaB* inactif, soit (ii) un chromosome dans lequel la séquence de nucléotides codant *RhaB* est supprimée.

15. Cellule hôte selon la revendication 13 ou 14, sachant que ladite cellule hôte est une cellule d'*Escherichia coli* W3110, de préférence comprenant un chromosome qui comprend une mutation du décalage du cadre de lecture dans la séquence de nucléotides codant RhaB.

16. Cellule hôte selon l'une quelconque des revendications 13 à 15, sachant que ladite cellule hôte est une cellule d'*Escherichia coli* W3110 comprenant un chromosome qui comprend la séquence de nucléotides de SEQ ID 2 ou une séquence ayant au moins 90 % d'identité de séquence avec celle-ci, de préférence la séquence de nucléotides de SEQ ID 2 ; sachant que facultativement, le chromosome de la cellule hôte comprend en outre une séquence de nucléotides codant RhaT.

17. Procédé de production de ranibizumab comprenant l'étape d'exposition de la cellule hôte bactérienne selon l'une quelconque des revendications 13 à 16 à du rhamnose, induisant ainsi l'expression de ranibizumab.

18. Procédé selon la revendication 17, comprenant en outre l'étape de récupération de ranibizumab à partir de la cellule hôte bactérienne ; facultativement comprenant en outre une ou plusieurs étapes de purification du ranibizumab récupéré, de préférence par une ou plusieurs étapes de chromatographie.
